(19) Europäisches Patentamt
European Patent Office
Office européen des brevets

(11) Publication number : 0 562 731 A1

# (12) EUROPEAN PATENT APPLICATION

(21) Application number : 93301705.5

(22) Date of filing : 05.03.93

(51) Int. Cl.[5] : C07D 401/12, A01N 47/36, C07D 213/76

(30) Priority : 10.03.92 JP 101549/92
24.07.92 JP 239931/92

(43) Date of publication of application :
29.09.93 Bulletin 93/39

(84) Designated Contracting States :
AT BE CH DE DK ES FR GB GR IE IT LI LU MC NL PT SE

(71) Applicant : ISHIHARA SANGYO KAISHA, Ltd.
3-22, Edobori 1-chome
Nishi-ku Osaka-shi Osaka-fu (JP)

(72) Inventor : Murai, Shigeo, c/o Ishihara Sangyo Kaisha Ltd.
Central Laboratory, 3-1, Nishishibukuwa 2-chome
Kusatsu-shi, Shiga (JP)
Inventor : Maeda, Kazuyuki, c/o Ishihara Sangyo Kaisha Ltd.
Central Laboratory, 3-1, Nishishibukuwa 2-chome
Kusatsu-shi, Shiga (JP)
Inventor : Nakamura, Yuji, c/o Ishihara Sangyo Kaisha Ltd.
Central Laboratory, 3-1, Nishishibukuwa 2-chome
Kusatsu-shi, Shiga (JP)
Inventor : Honzawa, Shooichi, c/o Ishihara Sangyo Kaisha Ltd.
Central Laboratory, 3-1, Nishishibukuwa 2-chome
Kusatsu-shi, Shiga (JP)
Inventor : Kanamori, Fumio, c/o Ishihara Sangyo Kaisha Ltd.
Central Laboratory, 3-1, Nishishibukuwa 2-chome
Kusatsu-shi, Shiga (JP)

(74) Representative : Pearce, Anthony Richmond
MARKS & CLERK Alpha Tower Suffolk Street
Queensway Birmingham B1 1TT (GB)

**(54) Substituted pyridinesulfonamide compounds or their salts, process for preparing the same, and herbicides containing the same.**

(57) A substituted pyridinesulfonamide compound or its salt represented by the following general formula (I) :

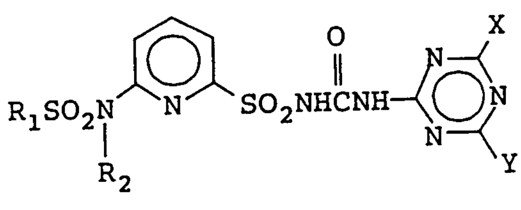

(I)

wherein $R_1$ is an unsubstituted or substituted alkyl group ; $R_2$ is an unsubstituted or substituted alkyl group, or an unsubstituted or substituted alkoxy group ; and X and Y are each independently a member selected from the group consisting of alkyl groups and alkoxy groups, is disclosed. This compound is useful as the effective ingredient of a herbicide showing a wide weed-control spectrum even if used in a small amount.

EP 0 562 731 A1

## FIELD OF THE INVENTION

The present invention relates to a novel substituted pyridinesulfonamide compound or its salt, a process for preparing the same, and a herbicide containing the same.

## BACKGROUND OF THE INVENTION

U.S. Patent No. 4,946,494 discloses a pyridinesulfonylurea derivative useful as an effective ingredient of a herbicidal composition, which is, however, different in the substituent at the 6-position of the pyridine ring in terms of chemical structure from the compound of the present invention.

EP 451,468 corresponding to U.S. Patent 5,139,565 and PCT WO91/10660 disclose a pyridinesulfonylurea derivative as an active ingredient of herbicidal compositions. This compound is different from the compound of the present invention in the position of an N-substituted sulfamoyl group.

## SUMMARY OF THE INVENTION

In accordance with one aspect of the present invention, there is provided a substituted pyridinesulfonamide compound or its salt represented by the following general formula (I):

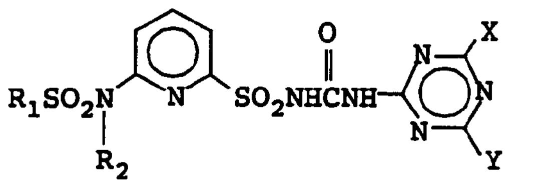

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups.

In accordance with another aspect of the present invention, there is provided a process for preparing a substituted pyridinesulfonamide compound or its salt represented by the following general formula (I):

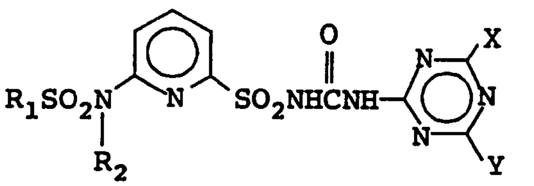

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups,
which comprises reacting a substituted pyridine compound represented by the following general formula (II):

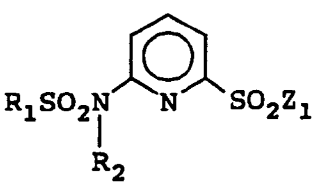

(II)

wherein $R_1$ and $R_2$ are the same as defined above; and $Z_1$ is a member selected from the group consisting of an $-NH_2$ group, an -NCO group, and $-NHCO_2R_3$ groups wherein $R_3$ is an alkyl or aryl group;
with a triazine compound represented by the following general formula (III):

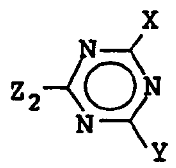

(III)

wherein X and Y are the same as defined above; and $Z_2$ is an $-NH_2$ group when $Z_1$ is an -NCO group or an $-NHCO_2R_3$ group, and is a member selected from the group consisting of an -NCO group and $-NHCO_2R_3$ groups wherein $R_3$ is the same as defined above, when $Z_1$ is an $-NH_2$ group.

In accordance with still another aspect of the present invention, there is provided a herbicide containing as the effective ingredient a substituted pyridinesulfonamide compound or its salt represented by the following general formula (I):

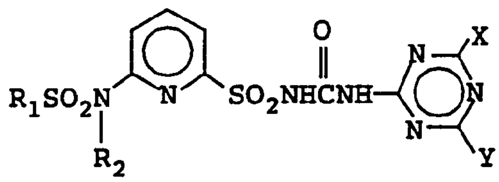

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups.

In accordance with a further aspect of the present invention, there is provided a compound represented by the following general formula:

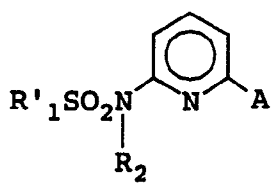

wherein $R'_1$ is an alkyl group substituted with at least one halogen atom; $R_2$ is an unsubstituted or substituted alkyl group, or an unsubstituted or substituted alkoxy group; and A is an $-SO_2NH_2$ group, or a

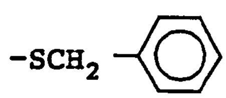

group.

## DETAILED DESCRIPTION OF THE INVENTION

The present invention will now be described in detail.

In the denotations of $R_1$ and $R_2$ in the general formula (I), the substituents that can be contained in the substituted alkyl group and the substituted alkoxy group include halogen atoms, alkoxy groups, etc. The number of substituent(s) contained in such a substituted group may be either one, or two or more, in which case the substituents may be the same or different from each other. The same applies to a substituent(s) if further contained in such a substituent as mentioned above that can be contained in such a substituted group that can be denoted by $R_1$ and $R_2$.

In the general formula (I), alkyl groups as well as alkyl moieties that may be included in the denotations of $R_1$, $R_2$, X and Y include those having 1 to 4 carbon atoms, such as methyl, ethyl, propyl and butyl groups that may each be linear or branched in terms of structural isomerism of the aliphatic chain. Halogen atoms

that may be included in the denotations of $R_1$ and $R_2$ include fluorine, chlorine, bromine and iodine atoms.

Examples of the salt of the substituted pyridinesulfonamide compound represented by the general formula (I) include those of alkali metals such as sodium and potassium, those of alkaline earth metals such as magnesium and calcium, and those of amines such as dimethylamine and triethylamine.

The substituted pyridinesulfonamide compounds represented by the general formula (I) or salts thereof may include their several optical isomers depending on the substituent(s) $R_1$ and/or $R_2$.

Among the compounds of the formula (I), preferred are those wherein X and Y are each independently a member selected from the group consisting of methyl group and methoxy group; more preferred are those wherein $R_1$ and $R_2$ may be either each independently alkyl groups which include those having 2 to 4 carbon atoms, and X and Y are each independently a member selected from the group consisting of methyl group and methoxy group; and most preferred are 6-[(N-ethyl-N-isopropylsulfonyl)amino]-N-[[(4-methoxy-6-methyltriazin-2-yl)amino]carbonyl]-2-pyridinesulfonamide (Compound No. 1 as described hereinafter), 6-[(N-ethyl-N-isopropylsulfonyl)amino]-N-[[(4,6-dimethoxytriazin-2-yl)amino]carbonyl]-2-pyridinesulfonamide (Compound No. 2 as described hereinafter), 6-[[N-(1-chloroethylsulfonyl)-N-ethyl]amino]-N-[[(4-methoxy-6-methyltriazin-2-yl)amino]carbonyl]-2-pyridinesulfonamide (Compound No. 54 as described hereinafter), 6-[[N-(1-chloroethylsulfonyl)-N-ethyl]amino]-N-[[(4,6-dimethoxytriazin-2-yl)amino]carbonyl]-2-pyridinesulfonamide (Compound No. 55 as described hereinafter) and 6-[(N-dichloromethylsulfonyl-N-ethyl)amino]-N-[[(4,6-dimethoxytriazine-2-yl)amino]carbonyl]-2-pyridinesulfonamide (Compound No. 57 as described hereinafter).

The substituted pyridinesulfonamide compound represented by the general formula (I) can be prepared, for example, according to the process of the present invention, embodiments of which may be represented by the following reaction formulae [A] to [D]:

Reaction Formula [A]

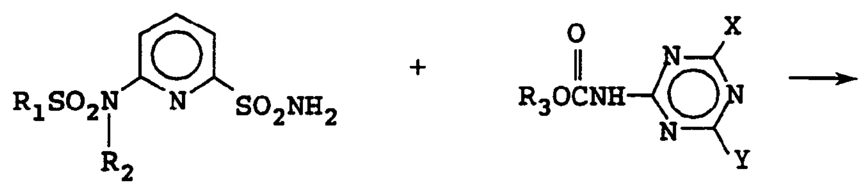

(I)

(II-1) (III-1)

Reaction Formula [B]

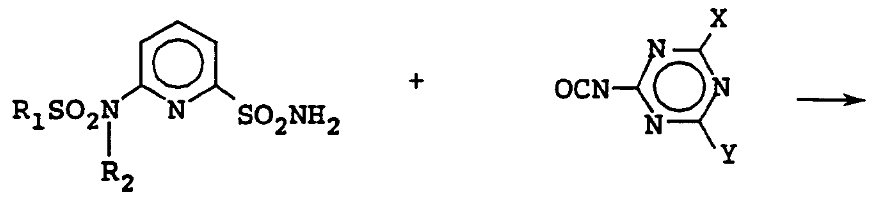

(I)

(II-1) (III-2)

Reaction Formula [C]

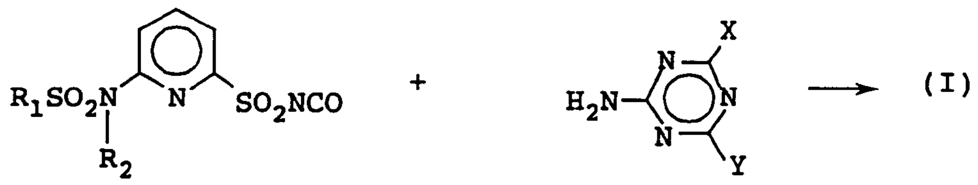

(II-2) (III-3)

Reaction Formula [D]

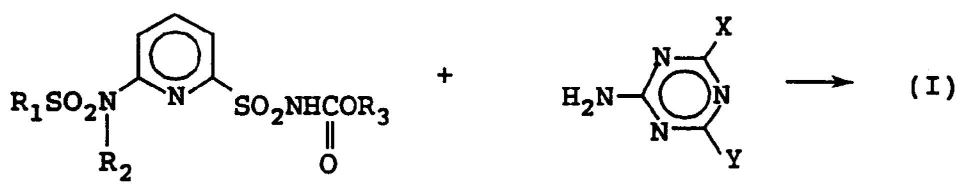

(II-3) (III-3)

In the reaction formulae [A] to [D], $R_1$, $R_2$, X and Y are the same defined above, and $R_3$ is an alkyl group or an aryl group.

The alkyl group represented by $R_3$ includes alkyl groups having from 1 to 6 carbon atoms, which may be substituted with a halogen atom, an alkoxy group, etc. As the aryl group included in the denotation of $R_3$, there can be mentioned a phenyl group, a phenyl group substituted with at least one chlorine atom, a phenyl group substituted with at least one methyl group, a naphthyl group, etc.

The reaction [A] is effected in the presence of a base, while the reactions [B], [C] and [D] may be effected in the presence of a base if desired. Examples of such a base include tertiary amines such as triethylamine, and 1,8-diazabicyclo[5.4.0]-7-undecene.

The reactions [A], [B], [C] and [D] may be effected in the presence of a solvent if necessary. Examples of the solvent include unsubstituted or substituted, aromatic hydrocarbons such as benzene, toluene, xylene, and chlorobenzene; unsubstituted or substituted, cyclic or acyclic, aliphatic hydrocarbons such as chloroform, carbon tetrachloride, methylene chloride, dichloroethane, trichloroethane, hexane, and cyclohexane; ethers such as diethyl ether, dioxane, and tetrahydrofuran; nitriles such as acetonitrile, propionitrile, and acrylonitrile; esters such as methyl acetate and ethyl acetate; aprotic polar solvents such as dimethyl sulfoxide and sulfolane; etc.

The reaction temperature of the reaction [A] is usually in the range of -20 to +100°C, preferably in the range of 0 to 40°C, while the reaction time of the reaction [A] is usually in the range of 0.01 to 24 hours, preferably in the range of 0.1 to 1.5 hours. The reaction temperature of the reaction [B] is usually in the range of 0 to 150°C, while the reaction time of the reaction [B] is usually in the range of 0.1 to 24 hours. The reaction temperature of the reaction [C] is usually in the range of 0 to 150°C, while the reaction time of the reaction [C] is usually in the range of 0.1 to 24 hours. The reaction temperature of the reaction [D] is usually in the range of -20 to +150°C, while the reaction time of the reaction [D] is usually in the range of 0.1 to 24 hours.

A starting material compound represented by the general formula (II-1) in the reaction formulae [A] and [B] according to the present invention can be synthesized, for example, according to one of reaction formulae [E], [F] and [G]:

Reaction Formula [E]

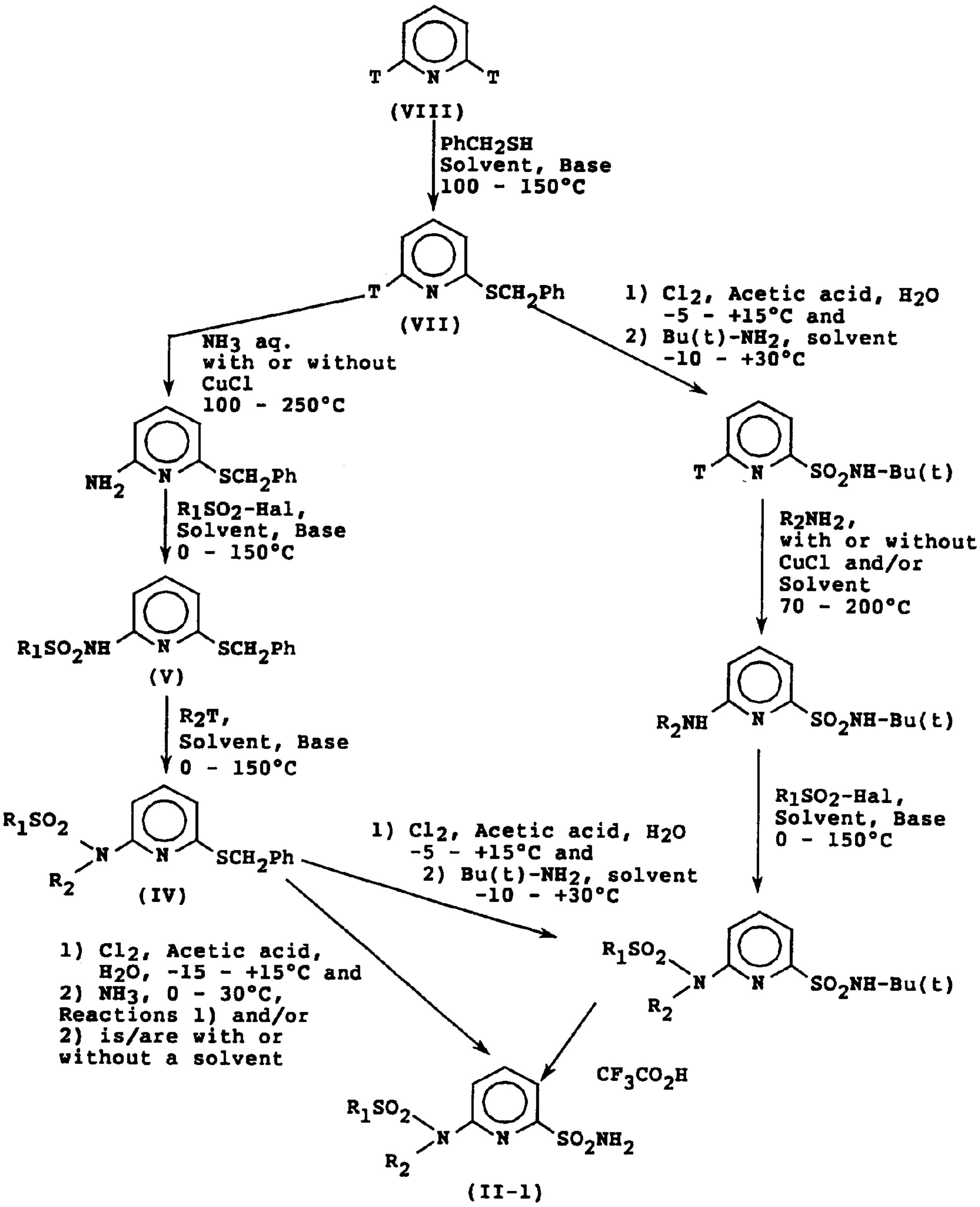

Reaction Formula [F]

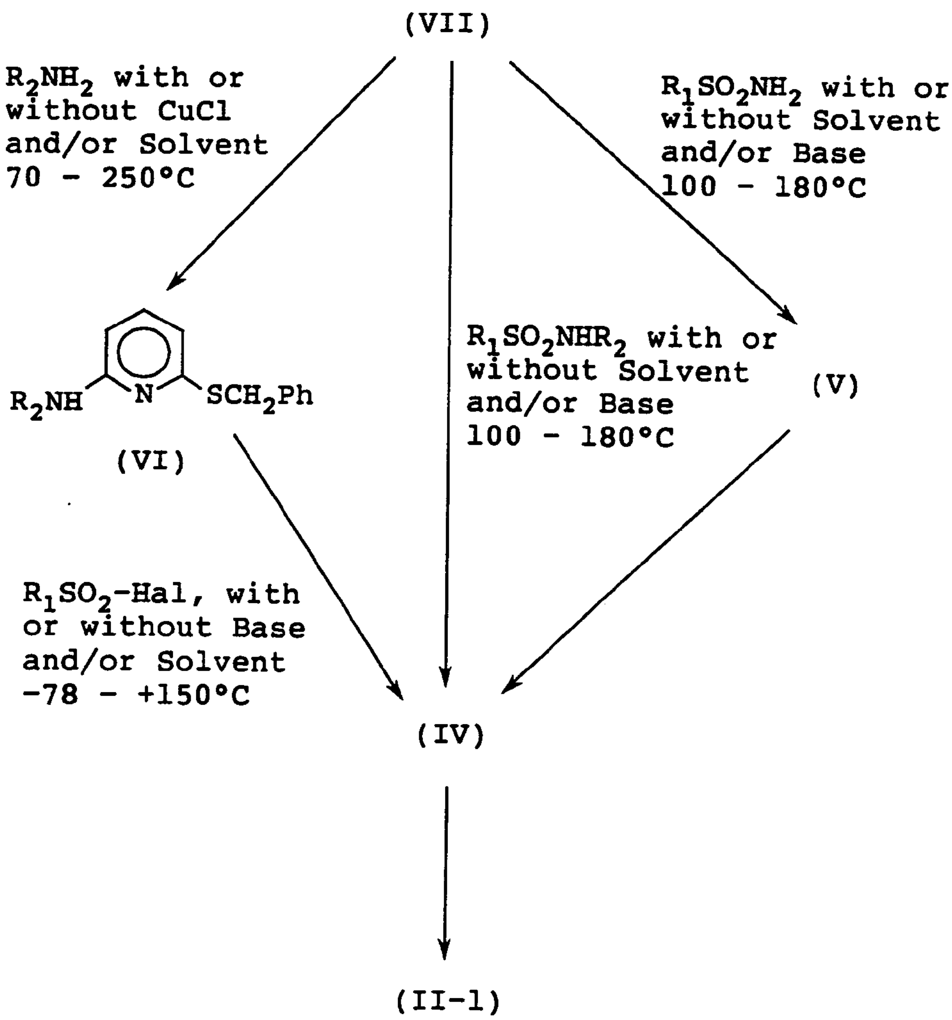

Reaction Formula [G]

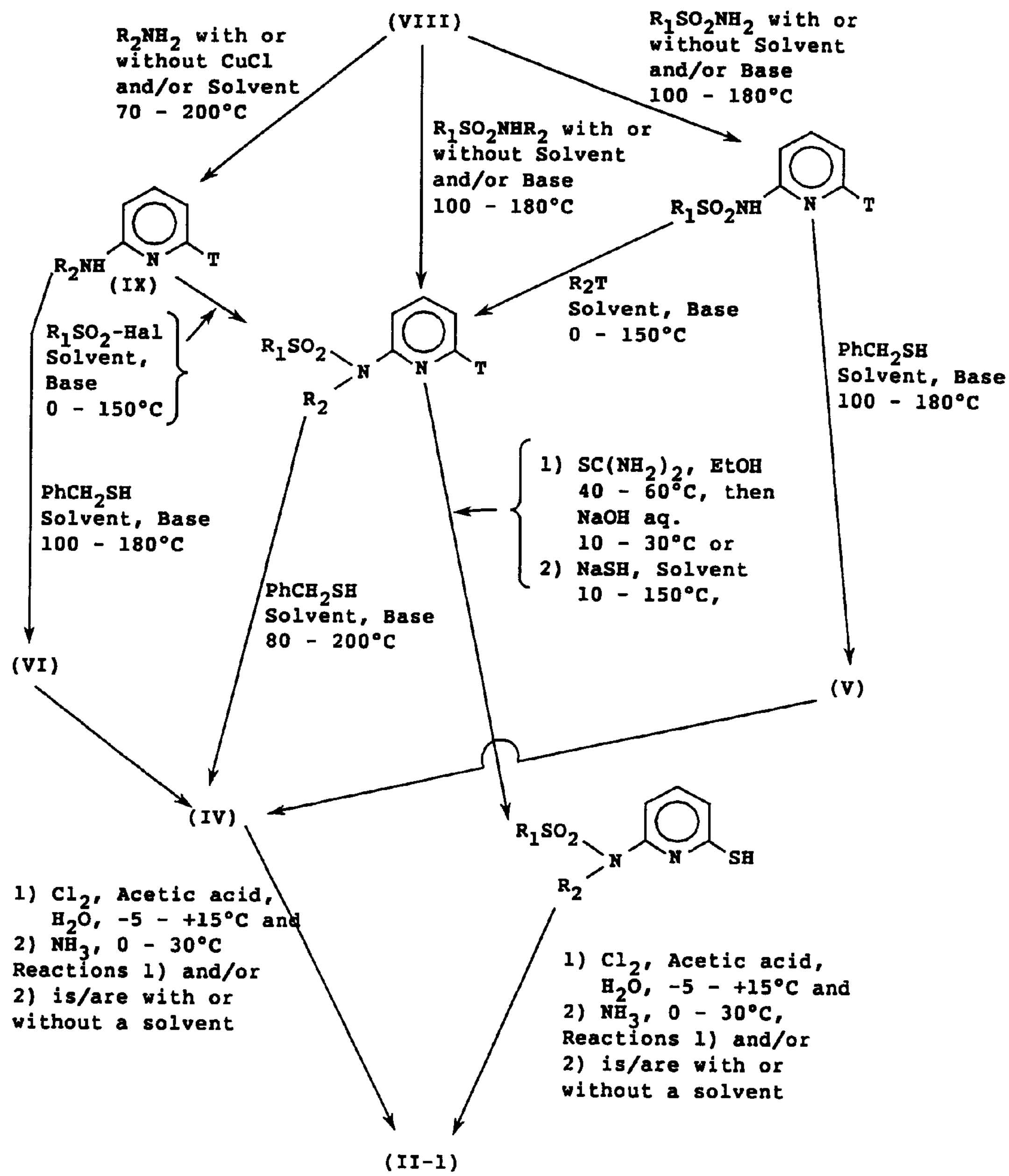


In the reaction formulae [E], [F] and [G], $R_1$ and $R_2$ are the same as defined above; Hal is a member selected from the group consisting of a fluorine atom, a chlorine atom, a bromine atom, and an iodine atom; T is a member selected from the group consisting of a chlorine atom, a bromine atom, and an iodine atom; Ph stands for a phenyl group; Et stands for an ethyl group; Bu(t) stands for a tertiary butyl group; and aq. stands for an aqueous solution.

The compound represented by the general formula (II-1) wherein $R_1$ is a halogen-substituted alkyl group may be prepared according to Reaction Formula H.

Reaction Formula H:

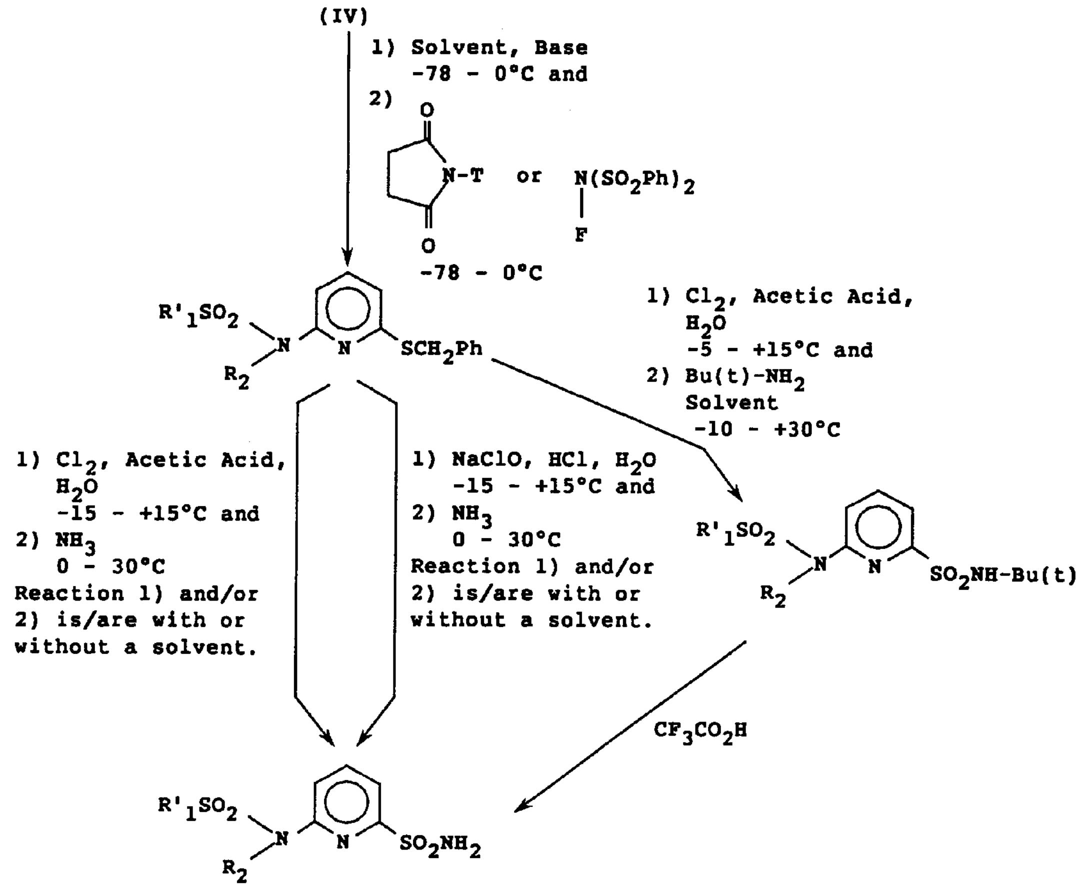


(II-1')

wherein $R'_1$ represents a halogen-substituted alkyl group; and $R_2$, T, Ph, and Bu(t) are as defined above.

In Reaction Formula G, the compound represented by formula (IX) may be prepared according to Reaction Formula I:

Reaction Formula I:

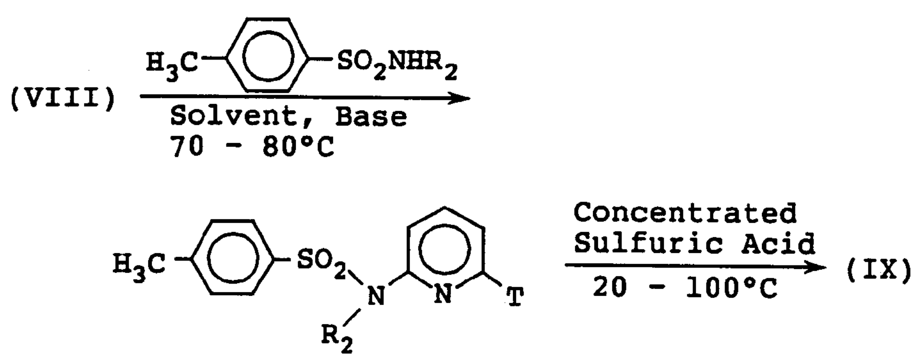

wherein $R_2$ and T are as defined above.

A starting material compound represented by the formula (II-2) in the reaction formula [C] can be prepared, for example, according to the following reaction formula [J]:

**Reaction Formula [J]**

$$(II\text{-}1) \xrightarrow[(2)\ COCl_2,\ xylene]{(1)\ CH_3(CH_2)_3NCO,\ K_2CO_3,\ CH_3COCH_3} (II\text{-}2)$$

A starting material compound represented by the formula (II-3) in the reaction formula [D] can be prepared, for example, according to the following reaction formula [K]:

**Reaction Formula [K]**

$$(II\text{-}1) \xrightarrow{ClCO_2R_3,\ NaH,\ tetrahydrofuran} (II\text{-}3)$$

$R_1$, $R_2$ and $R_3$ in the reaction formulae [J] and [K] are the same as defined hereinbefore.

The reaction conditions of the reactions [E] to [K], which involve the reaction temperature, the period of reaction time, the use or disuse as well as kind and amount of solvent (to be used if desired), and the kind and amount of base, can usually be appropriately chosen from the reaction conditions of similar reactions unless otherwise mentioned.

The salt of the aforementioned substituted pyridinesulfonamide compound can be easily prepared according to a usual method.

The following Examples will specifically illustrate the present invention in more detail, but should not be construed as limiting the scope of the invention.

## SYNTHESIS EXAMPLE 1

### Synthesis of 6-[(N-Ethyl-N-isopropylsulfonyl)amino]-N-[[(4-methoxy-6-methyltriazin-2-yl)amino]carbonyl-2-pyridinesulfonamide (Compound No. 1)

1) A mixture of 10 g of 2-benzylthio-6-bromopyridine, 70 ml of a 40% ethylamine aqueous solution, and a catalytic amount of cuprous chloride was allowed to react in an autoclave at 150°C for 10 hours. After completion of the reaction, the reaction mixture was poured into water and extracted with methylene chloride. The extract was washed with water, dried over anhydrous sodium sulfate, and concentrated under reduced pressure. The residue was purified by silica gel column chromatography (developing solvent: ethyl acetate/hexane = 1/4 by volume) to obtain 4.7 g of 2-benzylthio-6-ethylaminopyridine as an oily substance.

2) To a mixture of 3.16 g of the 2-benzylthio-6-ethylaminopyridine obtained in 1) above and 30 ml of tetrahydrofuran was added dropwise 9.4 ml of a 1.65 mol/ℓ hexane solution of n-butyl lithium at -10 to 0°C in a nitrogen stream. To the reaction mixture was added 1.84 g of isopropylsulfonyl chloride at 0 to 15°C, followed by reacting for 0.5 hour. After completion of the reaction, the reaction mixture was poured into water, made weakly acidic with hydrochloric acid, and extracted with methylene chloride. The extract was dried over anhydrous sodium sulfate, followed by concentration under reduced pressure. The residue was purified by silica gel column chromatography (developing solvent: ethyl acetate/hexane = 1/19 by volume) to obtain 0.77 g of N-(6-benzylthiopyridin-2-yl)-N-ethylisopropylsulfonamide as an oily substance.

3) A mixture of 0.75 g of the N-(6-benzylthiopyridin-2-yl)-N-ethylisopropylsulfonamide obtained in 2) above, 20 ml of acetic acid, and 15 ml of water was cooled to -5 to 0°C, and chlorine gas was introduced therein to conduct a reaction. After completion of the reaction, the reaction mixture was poured into water, extracted with methylene chloride, washed with water, and dried over anhydrous sodium sulfate. Ammonia gas was introduced into the reaction mixture to further conduct a reaction at room temperature for 1 hour.

After the reaction, the reaction mixture was filtered, and the filtrate was concentrated under reduced pressure. The residue was purified by silica gel column chromatography (developing solvent: ethyl acetate/hexane = 2/1 by volume) to obtain 0.50 g of 6-[(N-ethyl-N-isopropylsulfonyl)amino]-2-pyridinesulfonamide having a melting point of 107 to 109°C.

4) To a mixture of 0.10 g of 6-[(N-ethyl-N-isopropylsulfonyl)amino]-2-pyridinesulfonamide obtained in 3) above, 0.089 g of phenyl (4-methoxy-6-methyltriazin-2-yl)carbamate, and 7 ml of acetonitrile was added 0.050 g of 1,8-diazabicyclo[5.4.0]-7-undecene, and the mixture was allowed to react at room temperature for 1 hour. After completion of the reaction, the reaction mixture was poured into water and made weakly acidic with hydrochloric acid. The thus precipitated solid was collected by filtration, washed with water, and dried to obtain 0.13 g of the titled compound having a melting point of 146 to 148°C.

SYNTHESIS EXAMPLE 2

Synthesis of 6-[(N-Ethyl-N-isopropylsulfonyl)amino]-N-[[(4,6-dimethoxytriazin-2-yl)amino]carbonyl]-2-pyridinesulfonamide (Compound No. 2)

To a mixture of 0.15 g of the 6-[(N-ethyl-N-isopropylsulfonyl)amino]-2-pyridinesulfonamide obtained in Synthesis Example 1-3), 0.135 g of phenyl (4,6-dimethoxytriazin-2-yl)carbamate, and 7 ml of acetonitrile was added 0.074 g of 1,8-diazabicyclo[5.4.0]-7-undecene, and the mixture was allowed to react at room temperature for 1 hour. After the reaction, the reaction mixture was poured into water and made weakly acidic with hydrochloric acid. The thus precipitated solid was collected by filtration, washed with water, and dried to obtain 0.20 g of the titled compound having a melting point of 144 to 146°C.

SYNTHESIS EXAMPLE 3

Synthesis of 6-[[N-(1-Chloroethylsulfonyl)-N-ethyl]amino]-N-[[(4-methoxy-6-methyltriazin-2-yl)amino]carbonyl]-2-pyridinesulfonamide (Compound No. 54)

1) A mixture of 10 g of 2-benzylthio-6-bromopyridine, 70 ml of a 40% ethylamine aqueous solution, and a catalytic amount of cuprous chloride was allowed to react in an autoclave at 180°C for 4 hours. After completion of the reaction, the reaction mixture was poured into water and extracted with methylene chloride. The extract was washed with water, dried over anhydrous sodium sulfate, and concentrated under reduced pressure. The residue was purified by silica gel column chromatography (developing solvent: ethyl acetate/hexane = 1/19 by volume) to obtain 5.9 g of 2-benzylthio-6-ethylaminopyridine as an oily substance.

2) To a mixture of 3 g of the 2-benzylthio-6-ethylaminopyridine obtained in 1) above and 30 ml of tetrahydrofuran was added dropwise 8.1 ml of a 1.65 mol/ℓ hexane solution of n-butyl lithium at -10 to 0°C in a nitrogen stream. To the reaction mixture was added 1.9 g of ethylsulfonyl chloride at 0 to 15°C, followed by reacting for 0.5 hour. After completion of the reaction, the reaction mixture was poured into water and extracted with methylene chloride. The extract was dried over anhydrous sodium sulfate, followed by concentration under reduced pressure. The residue was purified by silica gel column chromatography (developing solvent: ethyl acetate/hexane = 1/19 by volume) to obtain 1.39 g of N-(6-benzylthiopyridin-2-yl)-N-ethyl(1-chloroethyl)sulfonamide having a melting point of 50 to 52°C.

3) A mixture of 1.39 g of the N-(6-benzylthiopyridin-2-yl)-N-ethyl(1-chloroethyl)sulfonamide obtained in 2) above, 30 ml of acetic acid, and 15 ml of water was cooled to -5 to 0°C, and chlorine gas was introduced therein to conduct a reaction. After completion of the reaction, the reaction mixture was poured into water, extracted with methylene chloride, washed with water, and dried over anhydrous sodium sulfate. Ammonia gas was introduced into the reaction mixture to further conduct a reaction at room temperature for 1 hour. After the reaction, the reaction mixture was filtered, and the filtrate was concentrated under reduced pressure. The residue was purified by silica gel column chromatography (developing solvent: ethyl acetate/hexane = 2/1 by volum) to obtain 1.11 g of 6-[[N-(1-chloroethylsulfonyl)-N-ethyl]amino]-2-pyridinesulfonamide having a melting point of 101 to 103°C.

4) To a mixture of 0.39 g of the 6-[[N-(1-chloroethylsulfonyl)-N-ethyl]amino]-2-pyridinesulfonamide obtained in 3) above, 0.31 g of phenyl (4-methoxy-6-methyltriazin-2-yl)carbamate, and 10 ml of acetonitrile was added 0.18 g of 1,8-diazabicyclo[5.4.0]-7-undecene, and the mixture was allowed to react at room temperature for 1 hour. After completion of the reaction, the reaction mixture was poured into water and made weakly acidic with hydrochloric acid. The thus precipitated solid was collected by filtration, washed with water, and dried to obtain 0.58 g of the desired compound having a melting point of 155 to 157°C. The product was found to be a racemic modification.

SYNTHESIS EXAMPLE 4

Synthesis of 6-[(N-Dichloromethylsulfonyl-N-ethyl)amino]-N-[[(4,6-dimethoxytriazin-2-yl)amino]carbonyl]-2-pyridinesulfonamide (Compound No. 57)

1) To a mixture of 6 g of 2-benzylthio-6-ethylaminopyridine and 60 ml of tetrahydrofuran was added dropwise 17.3 ml of a 1.65 mol/ℓ hexane solution of n-butyl lithium at -10 to 0°C in a nitrogen stream. To the reaction mixture was added 3.4 g of methysulfonyl chloride at 0 to 15°C, followed by reacting for 0.5 hour. After completion of the reaction, the reaction mixture was poured into water, made weakly acidic with hydrochloric acid, and extracted with methylene chloride. The extract was dried over anhydrous sodium sulfate, followed by concentration under reduced pressure. The residue was purified by silica gel column chromatography (developing solvent; ethyl acetate/hexane = 1/19 by volume) to obtain 3.4 g of N-(6-benzylthiopyridine-2-yl)-N-ethylmethylsulfonamide as an orange crystal.

$^1$H-NMR (60 MHz, $CDCl_3$) δ (ppm):

1.12 (3H, t, J=7.3Hz), 2.84 (3H, s), 3.85 (2H, q, J=7.3Hz), 4.34 (2H, s), 6.8-7.6 (8H, m)

2) To a mixture of 3.6 g of N-(6-benzylthiopyridin-2-yl)-N-ethylmethylsulfonamide and 50 ml of tetrahydrofuran was added dropwise 8 ml of a 1.65 mol/ℓ hexane solution of n-butyl lithium at -30 to -20°C in a nitrogen stream. Then, 1.8 g of N-chlorosuccinimide was added thereto at -30 to -20°C, followed by reacting for 1 hour. After completion of the reaction, the reaction mixture was poured into water and extracted with methylene chloride. The extract was dried over anhydrous sodium sulfate. followed by concentration under reduced pressure. The residue was purified by silica gel column chromatography (developing solvent: ethyl acetate/ hexane = 1/9 by volume) to obtain 0.7 g of N-(6-benzylthiopyridin-2-yl)-N-ethyldichloromethylsulfonamide as an oily substance.

$^1$H-NMR (60 MHz, $CDCl_3$) δ (ppm):

1.17 (3H, t, J=7.3Hz), 4.00 (2H, q, J=7.3Hz), 4.37 (2H, s), 6.53 (1H, s), 6,8-7.6 (8H, m)

3) A mixture of 1.4 g of N-(6-benzylthiopyridin-2-yl)-N-ethyldichloromethylsulfonamide, 20 ml of methylene chloride, 2.5 ml of concentrated hydrochloric acid, and 10 ml of water was cooled to -5 to 0°C, and 8 ml of a sodium hypochlorite solution (active chlorine: 8.5 to 13.5%) was added thereto dropwise to conduct a reaction. After completion of the reaction, the reaction mixture was poured into water, extracted with methylene chloride, washed with water, and dried over anhydrous sodium sulfate. Ammonia gas was introduced into the reaction mixture to futher conduct a reaction at room temperature for 1 hour. After the reaction, the reaction mixture was filtered, and the filtrate was concentrated under reduced pressure. The residue was purified by silica gel column chromatography (developing solvent: ethyl acetate/hexane = 2/1 by volume) to obtain 0.8 g of 6-[(N-dichloromethylsulfonyl-N-ethyl)amino]-2-pyridinesulfonamide having a melting point of 148 to 150°C.

4) To a mixture of 0.17 g of the 6-[(N-dichloromethylsulfonyl-N-ethyl)amino]-2-pyridinesulfonamide obtained in 3) above, 0.14 g of phenyl (4,6-dimethoxytriazin-2-yl)carbamate, and 10 ml of acetonitrile was added 0.08 g of 1,8-diazabicyclo[5.4.0]-7-undecene, and the mixture was allowed to react at room temperature for 1 hour. After completion of the reaction, the reaction mixture was poured into water and made weakly acidic with hydrochloric acid. The thus precipitated solid was collected by filtration, washed with water, and dried to obtain 0.18 g of the desired compound having a melting point of 157 to 159°C.

Of the intermediate compounds represented by the general formula (II-1) which can be synthesized by the above-mentioned process, those wherein $R_2$ is a substituted or unsubstituted alkoxy group are novel compounds. Typical examples of such novel compounds are shown in Table 1 below. Further, typical examples of the compounds represented by formula (I) which can be synthesized by the above-mentioned process are shown in Table 2 below.

TABLE 1

| Intermediate Compound No. | $R_1$ | $R_2$ | Physical Properties |
|---|---|---|---|
| 1 | $CH_3$ | $OCH_3$ | m.p. =93 to 96°C |
| 2 | $C_2H_5$ | $OCH_3$ | viscous oily substance |
| 3 | iso-$C_3H_7$ | $OCH_3$ | viscous oily substance |
| 4 | n-$C_4H_9$ | $OCH_3$ | |
| 5 | $CH_3$ | $OC_2H_5$ | |
| 6 | n-$C_3H_7$ | $OC_2H_5$ | |
| 7 | $CF_3$ | $OCH_3$ | |
| 8 | $CF_3$ | $OC_2H_5$ | |
| 9 | $CH_2CF_3$ | $OCH_3$ | |

TABLE 2

| Compound No. | $R_1$ | $R_2$ | X | Y | Melting Point (°C) |
|---|---|---|---|---|---|
| 1 | iso-$C_3H_7$ | $C_2H_5$ | $CH_3$ | $OCH_3$ | 146-148 |
| 2 | iso-$C_3H_7$ | $C_2H_5$ | $OCH_3$ | $OCH_3$ | 144-146 |
| 3 | $C_2H_5$ | n-$C_3H_7$ | $CH_3$ | $OCH_3$ | 152-154 |
| 4 | iso-$C_3H_7$ | n-$C_4H_9$ | $CH_3$ | $OCH_3$ | 162-164 |
| 5 | iso-$C_3H_7$ | n-$C_3H_7$ | $OCH_3$ | $OCH_3$ | 136-139 |
| 6 | $C_2H_5$ | iso-$C_3H_7$ | $CH_3$ | $OCH_3$ | 142-144 |
| 7 | $C_2H_5$ | iso-$C_3H_7$ | $OCH_3$ | $OCH_3$ | 142-143 |
| 8 | $C_2H_5$ | n-$C_3H_7$ | $OCH_3$ | $OCH_3$ | 151-154 |
| 9 | $C_2H_5$ | $C_2H_5$ | $OCH_3$ | $OCH_3$ | |
| 10 | $C_2H_5$ | n-$C_4H_9$ | $CH_3$ | $OCH_3$ | |
| 11 | $C_2H_5$ | n-$C_4H_9$ | $OCH_3$ | $OCH_3$ | |
| 12 | $C_2H_5$ | sec-$C_4H_9$ | $CH_3$ | $OCH_3$ | |
| 13 | $C_2H_5$ | sec-$C_4H_9$ | $OCH_3$ | $OCH_3$ | |
| 14 | n-$C_3H_7$ | $C_2H_5$ | $CH_3$ | $OCH_3$ | |
| 15 | n-$C_3H_7$ | $C_2H_5$ | $OCH_3$ | $OCH_3$ | |
| 16 | iso-$C_3H_7$ | n-$C_3H_7$ | $CH_3$ | $OCH_3$ | |
| 17 | iso-$C_3H_7$ | iso-$C_3H_7$ | $CH_3$ | $OCH_3$ | |
| 18 | iso-$C_3H_7$ | iso-$C_3H_7$ | $OCH_3$ | $OCH_3$ | |
| 19 | iso-$C_3H_7$ | n-$C_4H_9$ | $OCH_3$ | $OCH_3$ | |
| 20 | iso-$C_3H_7$ | sec-$C_4H_9$ | $CH_3$ | $OCH_3$ | |
| 21 | iso-$C_3H_7$ | sec-$C_4H_9$ | $OCH_3$ | $OCH_3$ | |
| 22 | n-$C_3H_7$ | n-$C_4H_9$ | $CH_3$ | $OCH_3$ | 159-161 |

TABLE 2 (continued)

| Compound No. | $R_1$ | $R_2$ | X | Y | Melting Point (°C) |
|---|---|---|---|---|---|
| 23 | n-$C_3H_7$ | n-$C_4H_9$ | $OCH_3$ | $OCH_3$ | |
| 24 | n-$C_3H_7$ | sec-$C_4H_9$ | $CH_3$ | $OCH_3$ | |
| 25 | n-$C_3H_7$ | sec-$C_4H_9$ | $OCH_3$ | $OCH_3$ | |
| 26 | n-$C_3H_7$ | n-$C_3H_7$ | $OCH_3$ | $OCH_3$ | |
| 27 | n-$C_3H_7$ | iso-$C_3H_7$ | $CH_3$ | $OCH_3$ | |
| 28 | n-$C_3H_7$ | iso-$C_3H_7$ | $OCH_3$ | $OCH_3$ | |
| 29 | sec-$C_4H_9$ | $C_2H_5$ | $CH_3$ | $OCH_3$ | |
| 30 | sec-$C_4H_9$ | $C_2H_5$ | $OCH_3$ | $OCH_3$ | |
| 31 | $CH_3$ | n-$C_3H_7$ | $CH_3$ | $OCH_3$ | 152-155 |
| 32 | $CH_3$ | n-$C_4H_9$ | $CH_3$ | $OCH_3$ | 162-167 |
| 33 | $CH_3$ | iso-$C_4H_9$ | $CH_3$ | $OCH_3$ | 145-148 |
| 34 | $CH_3$ | sec-$C_4H_9$ | $CH_3$ | $OCH_3$ | 160-162 |
| 35 | $CH_3$ | $CH_2OCH_3$ | $CH_3$ | $OCH_3$ | 155-159 |
| 36 | $C_2H_5$ | $CH_3$ | $CH_3$ | $OCH_3$ | 145-148 |
| 37 | $C_2H_5$ | $C_2H_5$ | $CH_3$ | $OCH_3$ | 154-157 |
| 38 | $C_2H_5$ | $CH_2OCH_3$ | $CH_3$ | $OCH_3$ | 153-158 |
| 39 | n-$C_3H_7$ | $CH_3$ | $CH_3$ | $OCH_3$ | 152-154 |
| 40 | n-$C_3H_7$ | n-$C_3H_7$ | $CH_3$ | $OCH_3$ | |
| 41 | iso-$C_3H_7$ | $OCH_3$ | $CH_3$ | $OCH_3$ | 141-143 |
| 42 | n-$C_4H_9$ | n-$C_3H_7$ | $CH_3$ | $OCH_3$ | 158-160 |
| 43 | n-$C_4H_9$ | n-$C_4H_9$ | $CH_3$ | $OCH_3$ | 157-159 |
| 44 | $CH_3$ | $OCH_3$ | $CH_3$ | $OCH_3$ | |

TABLE 2 (continued)

| Compound No. | $R_1$ | $R_2$ | X | Y | Melting Point (°C) |
|---|---|---|---|---|---|
| 45 | $C_2H_5$ | $OCH_3$ | $CH_3$ | $OCH_3$ | |
| 46 | iso-$C_3H_7$ | $OCH_3$ | $OCH_3$ | $OCH_3$ | |
| 47 | n-$C_4H_9$ | $OCH_3$ | $CH_3$ | $OCH_3$ | |
| 48 | $CH_3$ | $OC_2H_5$ | $CH_3$ | $OCH_3$ | |
| 49 | n-$C_3H_7$ | $OC_2H_5$ | $CH_3$ | $OCH_3$ | |
| 50 | $CF_3$ | $OCH_3$ | $CH_3$ | $OCH_3$ | |
| 51 | $CF_3$ | $OC_2H_5$ | $CH_3$ | $OCH_3$ | |
| 52 | $CH_2CF_3$ | $OCH_3$ | $CH_3$ | $OCH_3$ | |
| 53 | $CH_2CF_3$ | $CH_2OCH_3$ | $CH_3$ | $OCH_3$ | 137-144 |
| 54 | $CH_3CH(Cl)$ | $C_2H_5$ | $CH_3$ | $OCH_3$ | 155-157 |
| 55 | $CH_3CH(Cl)$ | $C_2H_5$ | $OCH_3$ | $OCH_3$ | 139-141 |
| 56 | $CHCl_2$ | $C_2H_5$ | $CH_3$ | $OCH_3$ | 155-157 |
| 57 | $CHCl_2$ | $C_2H_5$ | $OCH_3$ | $OCH_3$ | 157-159 |
| 58 | $CH_3CH(F)$ | $C_2H_5$ | $CH_3$ | $OCH_3$ | 136-141 |
| 59 | $CH_3CH(F)$ | $C_2H_5$ | $OCH_3$ | $OCH_3$ | |
| 60 | $CH_2Cl$ | $C_2H_5$ | $CH_3$ | $OCH_3$ | 172-174 |
| 61 | $CH_2Cl$ | $C_2H_5$ | $OCH_3$ | $OCH_3$ | |
| 62 | $CH_3CH(CF_3)$ | $C_2H_5$ | $CH_3$ | $OCH_3$ | |
| 63 | $CH_3CH(CF_3)$ | $C_2H_5$ | $OCH_3$ | $OCH_3$ | |
| 64 | $CH_3CH(CHF_2)$ | $C_2H_5$ | $CH_3$ | $OCH_3$ | |
| 65 | $CH_3CH(CHF_2)$ | $C_2H_5$ | $OCH_3$ | $OCH_3$ | |
| 66 | $CH_3CH(CH_2F)$ | $C_2H_5$ | $CH_3$ | $OCH_3$ | |

TABLE 2 (continued)

| Compound No. | $R_1$ | $R_2$ | X | Y | Melting Point (°C) |
|---|---|---|---|---|---|
| 67 | $CH_3CH(CH_2F)$ | $C_2H_5$ | $OCH_3$ | $OCH_3$ | |
| 68 | $CH_2Cl$ | $CH_3$ | $CH_3$ | $OCH_3$ | |
| 69 | $CH_2Cl$ | $CH_3$ | $OCH_3$ | $OCH_3$ | |
| 70 | $CH_2Cl$ | $n-C_3H_7$ | $CH_3$ | $OCH_3$ | |
| 71 | $CH_2Cl$ | $n-C_3H_7$ | $OCH_3$ | $OCH_3$ | |
| 72 | $CHF_2$ | $C_2H_5$ | $CH_3$ | $OCH_3$ | |
| 73 | $CH_2F$ | $C_2H_5$ | $CH_3$ | $OCH_3$ | |
| 74 | $CH_3CF_2$ | $C_2H_5$ | $CH_3$ | $OCH_3$ | |
| 75 | $CH_3CH(Cl)$ | $CH_2CF_3$ | $CH_3$ | $OCH_3$ | |
| 76 | $CH_3CH(Cl)$ | $CH_2CH_2F$ | $CH_3$ | $OCH_3$ | |
| 77 | $CH_3CH(Cl)$ | $CH_3CH_2Cl$ | $CH_3$ | $OCH_3$ | |
| 78 | $CH_3CH(Br)$ | $C_2H_5$ | $CH_3$ | $OCH_3$ | 157-159 |
| 79 | $CH_2CF_3$ | $C_2H_5$ | $CH_3$ | $OCH_3$ | 150-152 |
| 80 | iso-$C_4H_9$ | sec-$C_4H_9$ | $CH_3$ | $OCH_3$ | 85-90 |
| 81 | sec-$C_4H_9$ | sec-$C_4H_9$ | $CH_3$ | $OCH_3$ | 75-80 |
| 82 | n-$C_4H_9$ | iso-$C_4H_9$ | $CH_3$ | $OCH_3$ | oily substance |
| 83 | sec-$C_4H_9$ | iso-$C_4H_9$ | $CH_3$ | $OCH_3$ | oily substance |
| 84 | n-$C_4H_9$ | sec-$C_4H_9$ | $CH_3$ | $OCH_3$ | 65-69 |
| 85 | $CH_3CH(Cl)$ | $CH_3$ | $CH_3$ | $OCH_3$ | 161-163 |
| 86 | $CH_3CH(Cl)$ | $n-C_3H_7$ | $CH_3$ | $OCH_3$ | 159-160 |
| 87 | $(CH_3)_2CCl$ | $C_2H_5$ | $CH_3$ | $OCH_3$ | 145-146 |

TABLE 2 (continued)

| Compound No. | $R_1$ | $R_2$ | X | Y | Melting Point (°C) |
|---|---|---|---|---|---|
| 88 | $CH_3CH_2CH(Cl)$ | $C_2H_5$ | $CH_3$ | $OCH_3$ | 148-150 |
| 89 | $CBr_3$ | $CH_3$ | $CH_3$ | $OCH_3$ | 125-127 |
| 90 | $CBr_3$ | $C_2H_5$ | $CH_3$ | $OCH_3$ | 109-111 (decomposition) |
| 91 | $CCl_3$ | $C_2H_5$ | $CH_3$ | $OCH_3$ | 154-156 |
| 92 | $CH_2CF_3$ | $C_2H_5$ | $OCH_3$ | $OCH_3$ | 163-165 |
| 93 | $CH_3CH(Cl)$ | iso-$C_3H_7$ | $CH_3$ | $OCH_3$ | 148-151 |
| 94 | $(CH_2)_2OC_2H_5$ | $C_2H_5$ | $CH_3$ | $OCH_3$ | 124-127 |
| 95 | $CH_2CF_3$ | n-$C_3H_7$ | $CH_3$ | $OCH_3$ | 157-158 |
| 96 | $CF_3$ | $CH_2OCH_3$ | $CH_3$ | $OCH_3$ | 130-132 |
| 97 | $CH_3CH(Cl)$ | $CH_3$ | $CH_3$ | $CH_3$ | |
| 98 | $CH_3CH(Cl)$ | $C_2H_5$ | $CH_3$ | $CH_3$ | |
| 99 | $CH_3CH(Cl)$ | $C_2H_5$ | $CH_3$ | $OC_2H_5$ | |
| 100 | $CH_3CH(Cl)$ | $C_2H_5$ | $C_2H_5$ | $OCH_3$ | |
| 101 | $CH_3CH(OCH_3)$ | $CH_3$ | $CH_3$ | $OCH_3$ | |
| 102 | $CH_3CH(OCH_3)$ | $CH_3$ | $OCH_3$ | $OCH_3$ | |
| 103 | $CH_3CH(OCH_3)$ | $C_2H_5$ | $CH_3$ | $OCH_3$ | |
| 104 | $CH_3CH(OCH_3)$ | $C_2H_5$ | $OCH_3$ | $OCH_3$ | |
| 105 | $CH_3CH(OC_2H_5)$ | $CH_3$ | $CH_3$ | $OCH_3$ | |
| 106 | $CH_3CH(OC_2H_5)$ | $CH_3$ | $OCH_3$ | $OCH_3$ | |
| 107 | $CH_3CH(OC_2H_5)$ | $C_2H_5$ | $CH_3$ | $OCH_3$ | |
| 108 | $CH_3CH(OC_2H_5)$ | $C_2H_5$ | $OCH_3$ | $OCH_3$ | |

TABLE 2 (continued)

| Compound No. | $R_1$ | $R_2$ | X | Y | Melting Point (°C) |
|---|---|---|---|---|---|
| 109 | iso-$C_3H_7$ | $C_2H_5$ | $CH_3$ | $CH_3$ | |
| 110 | iso-$C_3H_7$ | $C_2H_5$ | $CH_3$ | $OC_2H_5$ | |
| 111 | Na salt of Compound No. 1 | | | | |
| 112 | Na salt of Compound No. 2 | | | | |
| 113 | Na salt of Compound No. 54 | | | | |
| 114 | Monomethylamine salt of Compound No. 54 | | | | |

In Table 2, the physical properties of Compound Nos. 34, 54, 55, 58, 78, 80, 81, 83 to 86, 88, and 93 are of the respective racemic modification.

As the effective ingredient of the herbicide, the substituted pyridinesulfonamide compound or its salt of the present invention, as will be evident from the test examples given hereinafter, exhibits a wide herbicidal spectrum at a low dosage while it shows safety on soybean, wheat, cotton, etc.

The herbicide of the present invention can be applied to a wide variety of sites including agricultural fields such as upland fields, orchards and mulberry fields, and non-agricultural fields such as forests, farm roads, playgrounds and factory sites. The method of application of the herbicide of the present invention can be arbitrarily chosen from a soil treatment application and a foliage treatment application. The herbicide of the present invention may be applied in the form of a formulation such as a dust, granules, water-dispersible granules, a wettable powder, an emulsifiable concentrate, a soluble concentrate, and a suspension concentrate, which is prepared by blending the substituted pyridinesulfonamide compound or its salt of the present invention as the effective ingredient usually with a carrier and, if necessary, further with various other adjuvants selected from among diluents, solvents, emulsifiers, spreaders, surfactants, etc. The suitable blending weight ratio of the effective ingredient to the agricultural adjuvants may be in the range of 1:99 to 90:10, preferably in the range of 5:95 to 80:20. The suitable amount of the effective ingredient to be used cannot be unequivocally determined because it varies depending on weather conditions, soil conditions, the form of the above-mentioned formulation, the kinds of object weeds, and the application season, however, the amount of the effective ingredient to be applied is generally in the range of 0.005 g to 50 g/a (are), preferably in the range of 0.01 to 10 g/a, more preferably in the range of 0.05 to 5 g/a.

The herbicide of the present invention may be used in mixture or combination with at least one ingredient or component selected from among other agricultural chemicals, agricultural adjuvants, phytotoxicity-reducing agents, etc. to sometimes exhibit improvements in effect and functions. The herbicide of the present invention may be used in mixture or combination with at least one other compound as an effective ingredient of herbicide, in which case a synergistic effect can sometimes be attained.

The other compound may be formulated together with the compound of the present invention, or its own preparation may be mixed with the herbicide of the present invention upon use.

Examples of the other compound are as follows:

Phenoxypropionate compounds such as

* ethyl (±)-2-[4-[(6-chloro-2-quinoxalinyl)oxy]phenoxy]propionate (common name: quizalofop-ethyl)
* ethyl (±)-2-[4-[(6-chloro-2-benzoxazolyl)oxy]phenoxy]propionate (common name: fenoxaprop-ethyl)
* butyl (±)-2-[4-[[5-(trifluoromethyl)-2-pyridinyl]oxy]phenoxy]propionate (common name: fluazifop-butyl)
* methyl 2-[4-[[3-chloro-5-(trifluoromethyl)-2-pyridinyl]oxy]phenoxy]propionate (common name: haloxyfop-methyl)
* 2-ethoxyethyl 2-[4-[[3-chloro-5-(trifluoromethyl)-2-pyridinyl]oxy]phenoxy]propionate (common name: haloxyfop-etotyl)
* (R)-2-[[(1-methylethylidene)amino]oxy]ethyl 2-[4-[6-chloro-2-quinoxalinyl)oxy]phenoxy]propanoate (common name: propaquizafop);

Diphenyl ether compounds such as
* sodium 5-[2-chloro-4-(trifluoromethyl)phenoxy]-2-nitrobenzoate (common name: acifluorfen-sodium)
* (±)-2-ethoxy-1-methyl-2-oxoethyl-5-[2-chloro-4-(trifluoromethyl)phenoxy]-2-nitrobenzoate (common name: lactofen)
* 5-[2-chloro-4-(trifluoromethyl)phenoxy]-N-(methylsulfonyl)-2-nitrobenzamide (common name: fomesafen);

Haloacetamide compounds such as
* 2-chloro-N-(2-ethyl-6-methylphenyl)-N-(2-methoxy-1-methylethyl)acetamide (common name: metolachlor)
* 2-chloro-N-(2,6-diethylphenyl)-N-(methoxymethyl)acetamide (common name: alachlor);
* N-[2,4-dimethyl-5-[[(trifluoromethyl)sulfonyl]amino]phenyl]acetamide (common name: mefluidide)

Imidazolinone compounds such as
* (±)-2-[4,5-dihydro-4-methyl-4-(1-methylethyl)-5-oxo-1H-imidazol-2-yl]-5-ethyl-3-pyridinecarboxylic acid (common name: imazethapyr)
* 2-[4,5-dihydro-4-methyl-4-(1-methylethyl)-5-oxo-1H-imidazol-2-yl]-3-quinolinecarboxylic acid (common name: imazaquin);

Dinitroaniline compounds such as
* 2,6-dinitro-N,N-dipropyl-4-(trifluoromethyl)aniline (common name: trifluralin)
* N-(1-ethylpropyl)-3,4-dimethyl-2,6-dinitroaniline (common name: pendimethalin);
* N-ethyl-α,α,α-trifluoro-N-(2-methylallyl)-2,6-dinitro-p-toluidine (common name: ethalfluralin)

Carbamate compounds such as
* 3-[(methoxycarbonyl)amino]phenyl (3-methylphenyl)carbamate (common name: phenmedipham)
* ethyl [3-[[(phenylamino)carbonyl]oxy]phenyl]carbamate (common name: desmedipham);
* S-ethyl dipropylcarbamothioate (common name: EPTC)
* S-propyl dipropylthiocarbamate (common name: vernolate)

Cyclohexane dione compounds such as
* 2-[1-(ethoxyimino)butyl]-5-[2-(ethylthio)propyl]-3-hydroxy-2-cyclohexen-1-one (common name: sethoxydim);

Sulfonylurea compounds such as
* ethyl 2-[[[[(4-chloro-6-methoxy-2-pyrimidinyl)amino]carbonyl]amino]sulfonyl]benzoate (common name: chlorimuron-ethyl)
* methyl 3-[[[[(4-methoxy-6-methyl-1,3,5-triazin-2-yl)amino]carbonyl]amino]sulfonyl]-2-thiophenecarboxylate (common name: thifensulfuron-methyl);

and

Compounds such as
* 3-(1-methylethyl)-(1H)-2,1,3-benzothiadiazin-4(3H)-one-2,2-dioxide (common name: bentazon)
* 4-(2,4-dichlorophenoxy)butanoic acid (common name: 2,4-DB)
* 3-(3,4-dichlorophenyl)-1-methoxy-1-methylurea (common name: linuron)
* 4-amino-6-(1,1-dimethylethyl)-3-(methylthio)-1,2,4-triazin-5(4H)-one (common name: metribuzin)
* 7-oxabicyclo[2.2.1]heptane-2,3-dicarboxylic acid (common name: endothal)
* (±)-2-ethoxy-2,3-dihydro-3,3-dimethyl-5-benzofuranyl methanesulfonate (common name: ethofumesate)
* 5-amino-4-chloro-2-phenyl-3(2H)-pyridazinone (Common name: chloridazon)
* 4-chloro-5-methylamino-2-(α,α,α-trifluoro-m-tolyl)pyridazin-3(2H)-one (common name: norflurazon)
* 2-(2-chlorobenzyl)-4,4-dimethyl-1,2-oxazolidin-3-one (common name: clomazone)

The amount of at least one compound selected from the above-exemplified other compound, based on the amount of the substituted pyridinesulfonamide compound or its salt as the effective ingredient of the herbicide of the present invention to be mixed therewith, may be in a comparatively wide range. However, the amount of the above-exemplified other compound is usually 0.002 to 1000 parts by weight, per part by weight of the substituted pyridinesulfonamide compound or its salt of the present invention.

In particular, the phenoxypropionate compounds, the imidazolinone compounds and the cyclohexane dione compounds are usually 0.04 to 100 parts by weight, the haloacetamide compounds and the dinitroaniline compounds are usually 1 to 600 parts by weight, the sulfonylurea compounds are usually 0.002 to 10 parts by weight, and a compound other than the above other compound is usually 0.02 to 800 parts by weight, per part by weight of the substituted pyridinesulfonamide compound or its salt of the present invention.

The substituted pyridinesulfonamide compound or its salt as the effective ingredient of the herbicide of the present invention may be used in an amount of 0.05 to 5 g/a in combination with at least one compound selected from the above-exemplified other compound in an amount of 0.01 to 50 g/a.

In particular, the phenoxypropionate compounds, the imidazolinone compounds and the cyclohexane dione compounds may be used in an amount of 0.2 to 5 g/a, the haloacetamide compounds and the dinitroaniline compounds may be used in an amount of 5 to 30 g/a, the sulfonylurea compounds may be used in an amount of 0.01 to 0.5 g/a, and the compound other than the above other compound may be further used in an amount of 0.1 to 40 g/a in combination with the substituted pyridinesulfonamide compound or its salt of the present invention.

Further, the following other compound as an effective ingredient of herbicide may be also used in mixture or combination with the herbicide of the present invention, where a synergistic effect can be attained, too.

Sulfonylurea compounds such as

* methyl 2-[[[[(4-methoxy-6-methyl-1,3,5-triazin-2-yl)amino]carbonyl]amino]sulfonyl]benzoate (common name: metsulfuron-methyl)
* methyl 2-[[[[N-(4-methoxy-6-methyl-1,3,5-triazin-2-yl)methylamino]carbonyl]amino]sulfonyl]benzoate (common name: tribenuron-methyl)
* 2-chloro-N-[[(4-methoxy-6-methyl-1,3,5-triazin-2-yl]amino]carbonyl]benzenesulfonamide (common name: chlorsulfuron);

Phenoxypropionate compounds such as

* methyl (±)-2-[4-(2,4-dichlorophenoxy)phenoxy]propionate (common name: diclofop-methyl);

Imidazolinone compounds such as

* mixture of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate (common name: imazamethabenz); and

Other compounds such as

* 1,2-dimethyl-3,5-diphenyl-1H-pyrazolium methylsulfate (common name: difenzoquat methylsulfate)
* 3,5-dibromo-4-hydroxybenzonitrile (common name: bromoxynil)
* 4-hydroxy-3,5-diiodobenzonitrile (common name: ioxynil)
* 2,4-dichlorophenoxyacetic acid (common name: 2,4-D)
* S-(2,3,3-trichloro-2-propenyl)-bis(1-methylethyl)carbamothioate (common name: triallate)
* 4-chloro-2-methylphenoxyacetic acid (common name: MCPA)
* 3,6-dichloro-2-pyridinecarboxylic acid (common name: clopyralid)
* O-(6-chloro-3-phenyl-4-pyridazinyl)-S-octyl thiocarbonothioate (common name: pyridate)
* 3,6-dichloro-2-methoxybenzoic acid (common name: dicamba)
* N'-(3,4-dichlorophenyl)-N,N-dimethylurea (common name: diuron)
* 4-amino-3,5,6-trichloro-2-pyridinecarboxylic acid (common name: picloram)
* N,N-dimethyl N'-[3-(trifluoromethyl)phenyl]urea (common name: fluometuron)
* 2-[[4-chloro-6-(ethylamino)-1,3,5-triazin-2-yl]amino]-2-methylpropionitrile (common name: cyanazine)
* N,N'-bis(1-methylethyl)-6-(methylthio)-1,3,5-triazine-2,4-diamine (common name: prometryn)
* N-(3,4-dichlorophenyl)propanamide (common name: propanil)
* disodium salt of methylarsonic acid (common name: DSMA)
* monosodium salt of methylarsonic acid (common name: MSMA)
* 2-chloro-1-(3-ethoxy-4-nitrophenoxy)-4-(trifluoromethyl)benzene (common name: oxyfluorfen)
* O,O-bis(1-methylethyl) S-[2-[(phenylsulfonyl)amino]ethyl]phosphorodithioate (common name: bensulide)
* 2-(3,4-dichlorophenyl)-4-methyl-1,2,4-oxadiazolidine-3,5-dione (common name: methazole)
* 4-chloro-5-(methylamino)-2-[3-(trifluoromethyl)phenyl]-3(2H)-pyridazinone (common name: norflurazon)
* 2-chloro-6-(4,6-dimethoxypyrimidin-2-ylthio)benzoate (common name: pyrithiobac)
* ethyl 2-chloro-6-(4,6-dimethoxypyrimidin-2-ylthio)benzoate (common name: pyrithiobac-ethyl)
* sodium 2-chloro-6-(4,6-dimethoxypyrimidin-2-ylthio)benzoate (common name: pyrithiobac-sodium)

The amount of at least one compound selected from the above-exemplified other compound, based on the amount of the substituted pyridinesulfonamide compound or its salt as the effective ingredient of the herbicide of the present invention to be mixed therewith, may be in a comparatively wide range. However, the amount of the above-exemplified other compound is usually 0.002 to 1000 parts by weight, per part by weight of the substituted pyridinesulfonamide compound or its salt of the present invention.

The substituted pyridinesulfonamide compound or its salt as the effective ingredient of the herbicide of the present invention may be used in an amount of 0.05 to 5 g/a in combination with at least one compound selected from the above-exemplified other compound in an amount of 0.01 to 50 g/a.

Test Examples, using the herbicide of the present invention, will now be described.

Test Example 1

1/1,500 are [are (a) = 100 $m^2$] pots were filled with upland farming soil, which in the pots was then sown with a variety of plants. The plants grown will be enumerated below.

| Name | Abbreviation |
|---|---|
| rice (Oryza sativa) | OR |
| soybean (Glycine max) | GL |
| corn (Zea mays) | ZE |
| cotton (Gossypium) | GO |
| wheat (Triticum) | TR |
| cocklebur (Xanthium strumarium) | XA |
| morning glory (Ipomoea purpurea) | IP |
| prickly sida (Sida spinosa) | SI |
| slender amaranth (Amaranthus viridis) | AM |
| barnyard grass (Echinochloa crus-galli) | EC |
| large crabgrass (Digitaria adscendens) | DI |

When each plant reached a given growth stage (a 1.2- to 3.0-leaf stage for rice, primary leaf to a 0.7-leaf stage for soybean, 2.1- to 3.6-leaf stage for corn, cotyledon to 0.7-leaf stage for cotton, 2.0- to 3.0-leaf stage for wheat, 0.3- to 3.4-leaf stage for cocklebur, 0.3- to 1.6-leaf stage for morning glory, 0.1- to 1.3-leaf stage for prickly sida, 0.1- to 1.3-leaf stage for slender amaranth, 1.5- to 3.2-leaf stage for barnyard glass, and 1.2- to 3.0-leaf stage for large crabgrass), a predetermined amount of the herbicide of the present invention in the form of a wettable powder, weighed out, diluted with 5 ℓ/a of water and admixed with 0.2 or 0.1 vol%, based on the resulting composition, of an agricultural spreader, was foliarly applied to the plant with a small spray gun. The progress of growth of the plant was visually observed 20 to 31 days after the foliar application to evaluate the degree of growth control according to 10 ratings (1: the same as in an untreated plot - 10: perfect growth control). The results are listed in Table 3.

TABLE 3

| Compound No. | Amount of Active Ingredient (g/a) | OR | GL | ZE | GO | TR | XA | IP | SI | AM | EC | DI | Day* |
|---|---|---|---|---|---|---|---|---|---|---|---|---|---|
| 1 | 1.25 | 10 | 4 | 10 | | 8 | 10 | 9 | 9 | 10 | 10 | 8 | 26 |
| | 0.31 | 9 | 3 | 9 | | 8 | 9 | 8 | 8 | 10 | 9 | 8 | |
| 2 | 1.25 | 10 | 3 | 10 | | 10 | 10 | 10 | 6 | 10 | 9 | 9 | 21 |
| | 0.31 | 8 | 2 | 9 | | 8 | 10 | 9 | 5 | 10 | 8 | 8 | |
| 3 | 1.25 | 6 | 3 | 8 | | 4 | 8 | 5 | 6 | 10 | 9 | 6 | 27 |
| 4 | 1.25 | 8 | 4 | 10 | | 2 | 9 | 6 | 6 | 9 | 7 | 3 | 31 |
| 5 | 1.25 | 10 | 4 | 10 | | 10 | 10 | 9 | 5 | 10 | 10 | 6 | 21 |
| | 0.31 | 9 | 3 | 7 | | 9 | 10 | 8 | 4 | 9 | 10 | 5 | |
| 6 | 1.25 | 6 | 3 | 10 | | 3 | 7 | 8 | 6 | 9 | 6 | 4 | 22 |
| 7 | 1.25 | 3 | 3 | 10 | | 2 | 7 | 6 | 3 | 9 | 2 | 2 | 22 |
| 8 | 1.25 | 3 | 4 | 5 | | 3 | 6 | 4 | 3 | 7 | 5 | 5 | 23 |
| 32 | 1.25 | 3 | 3 | 3 | | 2 | 6 | 5 | 4 | 6 | 3 | 6 | 27 |
| 33 | 1.25 | 6 | 3 | 2 | | 3 | 3 | 4 | 3 | 6 | 3 | 6 | 32 |
| 37 | 1.25 | 2 | 2 | 3 | | 1 | 4 | 3 | 3 | 9 | 4 | 2 | 31 |
| 39 | 1.25 | 3 | 4 | 3 | | 3 | 5 | 5 | 3 | 9 | 3 | 4 | 21 |
| 41 | 1.25 | 10 | 2 | 10 | 3 | | 8 | 6 | 5 | 9 | 10 | 9 | 20 |
| | 0.31 | 9 | 1 | 10 | 1 | | 6 | 5 | 2 | 7 | 10 | 7 | |

TABLE 3 (continued)

| Compound No. | Amount of Active Ingredient (g/a) | OR | GL | ZE | GO | TR | XA | IP | SI | AM | EC | DI | Day* |
|---|---|---|---|---|---|---|---|---|---|---|---|---|---|
| 42 | 1.25 | 6 | 4 | 3 | | 4 | 6 | 4 | 3 | 10 | 3 | 5 | 21 |
| 43 | 1.25 | 2 | 4 | 3 | | 3 | 6 | 3 | 3 | 5 | 3 | 6 | 21 |
| 54 | 1.25 | 9 | 2 | 10 | 6 | | 10 | 9 | 7 | 9 | 10 | 3 | 21 |
| | 0.31 | 9 | 1 | 10 | 4 | | 9 | 9 | 6 | 9 | 10 | 3 | |
| 55 | 1.25 | 9 | 3 | 10 | 7 | | 10 | 9 | 6 | 6 | 10 | 7 | 23 |
| | 0.31 | 9 | 2 | 10 | 5 | | 10 | 8 | 6 | 6 | 10 | 5 | |
| 56 | 1.25 | 9 | 4 | 10 | | 10 | 10 | 10 | 8 | 10 | 10 | 10 | 24 |
| | 0.31 | 9 | 3 | 10 | | 10 | 10 | 9 | 6 | 9 | 10 | 10 | |
| 57 | 1.25 | 8 | 2 | 9 | | 6 | 10 | 10 | 8 | 9 | 8 | 9 | 25 |
| | 0.31 | 6 | 1 | 6 | | 6 | 10 | 10 | 7 | 8 | 7 | 9 | |
| 58 | 1.25 | 5 | 1 | 4 | | 2 | 8 | 6 | 7 | 8 | 6 | 7 | 21 |
| | 0.31 | 4 | 1 | 3 | | 2 | 7 | 5 | 6 | 7 | 5 | 7 | |
| 60 | 1.25 | 6 | 4 | 5 | | 2 | 9 | 7 | 5 | 9 | 6 | 4 | 21 |
| 78 | 1.25 | 10 | 10 | 10 | 9 | | 10 | 10 | 6 | 4 | 10 | 8 | 20 |
| | 0.31 | 9 | 6 | 10 | 8 | | 10 | 10 | 5 | 3 | 10 | 6 | |
| 79 | 1.25 | 1 | 6 | 1 | 6 | | 6 | 7 | 3 | 6 | 1 | 3 | 31 |
| 80 | 1.25 | 3 | 6 | 2 | | 4 | 6 | 5 | 3 | 7 | 4 | 2 | |
| | 0.31 | 1 | 5 | 1 | | 3 | 6 | 5 | 3 | 6 | 3 | 2 | 21 |
| 81 | 1.25 | 5 | 6 | 2 | | 2 | 9 | 6 | 2 | 3 | 6 | 3 | 21 |

TABLE 3 (continued)

| Compound No. | Amount of Active Ingredient (g/a) | OR | GL | ZE | GO | TR | XA | IP | SI | AM | EC | DI | Day* |
|---|---|---|---|---|---|---|---|---|---|---|---|---|---|
| 82 | 1.25 | 4 | 3 | 8 | | 4 | 9 | 6 | 1 | 7 | 4 | 3 | 22 |
| 84 | 1.25 | 6 | 6 | 3 | | 6 | 7 | 6 | 3 | 8 | 5 | 4 | 21 |
| | 0.31 | 5 | 5 | 2 | | 5 | 6 | 6 | 2 | 7 | 5 | 3 | |
| 85 | 1.25 | 10 | 5 | 10 | 6 | | 10 | 10 | 6 | 7 | 10 | 6 | 20 |
| | 0.31 | 9 | 2 | 10 | 3 | | 9 | 7 | 4 | 5 | 10 | 5 | |
| 86 | 1.25 | 10 | 10 | 10 | 9 | | 10 | 10 | 5 | 6 | 10 | 6 | 20 |
| | 0.31 | 9 | 6 | 10 | 8 | | 9 | 10 | 4 | 5 | 10 | 3 | |
| 87 | 1.25 | 8 | 6 | 9 | 6 | | 10 | 10 | 4 | 6 | 5 | 3 | 20 |
| | 0.31 | 6 | 3 | 8 | 3 | | 10 | 6 | 2 | 4 | 3 | 2 | |
| 88 | 1.25 | 10 | 6 | 10 | 6 | | 10 | 10 | 6 | 6 | 10 | 6 | 22 |
| | 0.31 | 9 | 5 | 10 | 6 | | 10 | 10 | 5 | 5 | 10 | 5 | |
| 89 | 1.25 | 9 | 9 | 9 | 5 | | 10 | 10 | 5 | 7 | 10 | 7 | 21 |
| | 0.31 | 6 | 5 | 6 | 4 | | 10 | 8 | 3 | 6 | 9 | 7 | |
| 90 | 1.25 | 9 | 10 | 8 | 7 | | 10 | 9 | 6 | 7 | 10 | 6 | 21 |
| | 0.31 | 8 | 9 | 7 | 5 | | 10 | 8 | 6 | 6 | 10 | 6 | |
| 91 | 1.25 | 9 | 10 | 10 | | 9 | 10 | 9 | 9 | 10 | 10 | 9 | 21 |
| | 0.31 | 9 | 9 | 9 | | 9 | 10 | 9 | 7 | 9 | 9 | 6 | |
| 92 | 1.25 | 5 | 7 | 7 | | 9 | 10 | 10 | 6 | 5 | 6 | 3 | 20 |
| 93 | 1.25 | 9 | 7 | 9 | | 10 | 10 | 9 | 7 | 9 | 10 | 7 | 22 |
| | 0.31 | 9 | 6 | 8 | | 9 | 10 | 9 | 5 | 9 | 7 | 5 | |

TABLE 3 (continued)

| Compound No. | Amount of Active Ingredient (g/a) | OR | GL | ZE | GO | TR | XA | IP | SI | AM | EC | DI | Day* |
|---|---|---|---|---|---|---|---|---|---|---|---|---|---|
| 94 | 1.25 | 4 | 6 | 7 | 7 | | 9 | 7 | 6 | 9 | 9 | 3 | 21 |
| | 0.31 | 3 | 3 | 4 | 6 | | 8 | 4 | 4 | 6 | 4 | 2 | |
| 95 | 1.25 | 6 | 10 | 10 | 9 | | 10 | 9 | 6 | 6 | 6 | 5 | 20 |
| | 0.31 | 5 | 9 | 9 | 8 | | 9 | 8 | 5 | 5 | 3 | 4 | |

Note:* The day of observation after foliar application.

TEST EXAMPLE 2

1/10000-are pot was filled with upland farming soil and sown with velvet leaf (Abutilon theophrasti). When

the plant reached the 1.2-leaf stage, a predetermined amount of the herbicide of the present invention diluted with 5 ℓ of water per are and admixed with 0.1 vol%, based on the aqueous solution, of an agricultural spreader ("Kusa Rino", produced by Nihon Nohyaku Co., Ltd.) was foliarly applied to the plant with a small spray gun. The herbicidal effect was visually observed 25 days after the foliar application and evaluated according to the rating system shown below. The results obtained are shown in Table 4.

Rating System:

| Rating | Precent Growth Inhibition (%) |
|---|---|
| 1 | 0-19 |
| 2 | 20-29 |
| 3 | 30-39 |
| 4 | 40-49 |
| 5 | 50-59 |
| 6 | 60-69 |
| 7 | 70-79 |
| 8 | 80-89 |
| 9 | 90-99 |
| 10 | 100 |

TABLE 4

| Active Ingredient | Amount of Active Ingredient (g/a) | Rating |
|---|---|---|
| Compound No. 54 | 1.25 | 8 |
| " | 0.63 | 6 |
| " | 0.31 | 5 |
| " | 0.16 | 3 |
| Chlorimuron-ethyl | 0.08 | 9 |
| " | 0.04 | 8 |
| " | 0.02 | 6 |
| Fomesafen | 1.00 | 7 |
| " | 0.5 | 7 |
| " | 0.25 | 3 |
| Compound No. 54 + Chlorimuron-ethyl | 1.25 + 0.08 | 10 |
| " | 1.25 + 0.04 | 10 |
| " | 1.25 + 0.02 | 10 |
| " | 0.63 + 0.08 | 10 |
| " | 0.63 + 0.04 | 10 |
| " | 0.63 + 0.02 | 10 |
| " | 0.31 + 0.08 | 10 |
| " | 0.31 + 0.04 | 9 |
| " | 0.31 + 0.02 | 9 |
| " | 0.16 + 0.08 | 10 |
| " | 0.16 + 0.04 | 9 |

TABLE 4 (Cont'd.)

| Active Ingredient | Amount of Active Ingredient (g/a) | Rating |
|---|---|---|
| Compound No. 54 + Chlorimuron-ethyl | 0.16 + 0.02 | 8 |
| Compound No. 54 + Fomesafen | 1.25 + 1.00 | 10 |
| " | 1.25 + 0.5 | 10 |
| " | 1.25 + 0.25 | 10 |
| " | 0.63 + 1.00 | 10 |
| " | 0.63 + 0.5 | 10 |
| " | 0.63 + 0.25 | 9 |
| " | 0.31 + 1.00 | 10 |
| " | 0.31 + 0.5 | 10 |
| " | 0.31 + 0.25 | 8 |
| " | 0.16 + 1.00 | 10 |
| " | 0.16 + 0.5 | 9 |
| " | 0.16 + 0.25 | 7 |

TEST EXAMPLE 3

1/10000-are pot was filled with upland farming soil and sown with velvet leaf. When the plant reached the 1-leaf stage, a predetermined amount of the herbicide of the present invention diluted with 5 ℓ of water per are and admixed with 0.1 vol%, based on the aqueous solution, of an agricultural spreader ("Kusa Rino") was foliarly applied to the plant with a small spray gun. On the 20th day from the application, the aerial part of the plant was weighed to obtain a percent growth inhibition according to Equation (1) (found value) and a Colby's value (calculated value) according to Equation (2) (see Weed, Vol. 15, pp. 20-22 (1967)). The results obtained are shown in Table 5 below.

$$\text{Percent Growth Inhibition (\%)} = \left(1 - \frac{\text{Fresh Weight of Aerial Part of Plant in Treated Plot}}{\text{Fresh Weight of Aerial Part of Plant in Untreated Plot}}\right) \times 100 \qquad (1)$$

$$\text{Colby's Value (E)} = \alpha + \beta - \frac{\alpha \times \beta}{100} \qquad (2)$$

Wherein $\alpha$ represents a percent growth inhibition when treated with an amount a (g/a) of herbicide A; $\beta$ represents a percent growth inhibition when treated with amount b (g/a) of herbicide B; and E represents an expected percent growth inhibition when treated with a of herbicide A and b of herbicide B.

That is, if a percent growth inhibition (found value) is higher than a Colby's value (calculated value) by the above-mentioned calculation, it can be said that the activity brought by the combination of herbicides exhibits a synergistic action.

TABLE 5

| Active Ingredient | Amount of Active Ingredient | Percent Growth Inhibition (%) | |
|---|---|---|---|
| | (g/a) | Found | Calcd. |
| Compound No. 54 | 0.63 | 86 | |
| " | 0.31 | 52 | |
| " | 0.16 | 19 | |
| Bentazon | 1.25 | 69 | |
| " | 0.63 | 17 | |
| Metribuzin | 0.16 | 89 | |
| Compound No. 54 + Benzaton | 0.63 + 1.25 | 100 | 96 |
| " | 0.63 + 0.63 | 93 | 88 |
| " | 0.31 + 1.25 | 100 | 85 |
| " | 0.31 + 0.63 | 94 | 60 |
| " | 0.16 + 1.25 | 90 | 75 |
| " | 0.16 + 0.63 | 50 | 33 |
| Compound No. 54 + Metribuzin | 0.63 + 0.16 | 100 | 98 |
| " | 0.31 + 0.16 | 100 | 95 |
| " | 0.16 + 0.16 | 100 | 91 |

TEST EXAMPLE 4

1/10000-are pot was filled with upland farming soil and sown with velvet leaf. When the plant reached the 1-leaf stage, a predetermined amount of the herbicide of the present invention diluted with 5 ℓ of water per are and admixed with 0.1 vol%, based on the aqueous solution, of an agricultural spreader ("Kusa Rino") was foliarly applied to the plant with a small spray gun. On the 20th day from the application, the aerial part of the plant was weighed to obtain a percent growth inhibition and a Colby's value in the same manner as in Test Example 3. The results obtained are shown in Table 6 below.

TABLE 6

| Active Ingredient | Amount of Active Ingredient (g/a) | Percent Growth Inhibition (%) | |
|---|---|---|---|
| | | Found | Calcd. |
| Compound No. 54 | 0.63 | 66 | |
| " | 0.31 | 32 | |
| " | 0.16 | 12 | |
| Linuron | 0.25 | 61 | |
| " | 0.125 | 35 | |

TABLE 6 (Cont'd.)

| Active Ingredient | Amount of Active Ingredient (g/a) | Percent Growth Inhibition (%) Found | Calcd. |
|---|---|---|---|
| Compound No. 54 + Linuron | 0.63 + 0.25 | 91 | 87 |
| " | 0.63 + 0.125 | 84 | 78 |
| " | 0.31 + 0.25 | 74 | 73 |
| " | 0.31 + 0.125 | 69 | 56 |
| " | 0.16 + 0.25 | 74 | 66 |
| " | 0.16 + 0.125 | 55 | 43 |

TEST EXAMPLE 5

1/10000-are pot was filled with upland farming soil and sown with large crabgrass (Digitaria adscendens). When the plant reached the 2-leaf stage, a predetermined amount of the herbicide of the present invention diluted with 5 ℓ of water per are and admixed with 0.1 vol%, based on the aqueous solution, of an agricultural spreader ("Kusa Rino") was foliarly applied to the plant with a small spray gun. On the 20th day from the application, the aerial part of the plant was weighed to obtain a percent growth inhibition and a Colby's value in the same manner as in Test Example 3. The results obtained are shown in Table 7 below.

TABLE 7

| Active Ingredient | Amount of Active Ingredient | Percent Growth Inhibition (%) | |
|---|---|---|---|
| | (g/a) | Found | Calcd. |
| Compound No. 54 | 0.63 | 77 | |
| " | 0.31 | 24 | |
| " | 0.16 | 19 | |
| Linuron | 0.5 | 79 | |
| " | 0.25 | 68 | |
| " | 0.125 | 23 | |
| Compound No. 54 + Linuron | 0.63 + 0.5 | 98 | 95 |
| " | 0.63 + 0.25 | 98 | 93 |
| " | 0.63 + 0.125 | 97 | 82 |
| " | 0.31 + 0.5 | 96 | 84 |
| " | 0.31 + 0.25 | 91 | 76 |
| " | 0.31 + 0.125 | 87 | 41 |
| " | 0.16 + 0.5 | 89 | 83 |
| " | 0.16 + 0.25 | 78 | 74 |
| " | 0.16 + 0.125 | 41 | 38 |

Formulation Examples of the herbicide of the present invention will now be described.

Formulation Example 1

| | |
|---|---|
| (1) clay (trade name: Newlite) | 97 parts by weight |
| (2) a polyoxyethylene octylphenyl ether premixed with white carbon (trade name: Dikssol W-92 | 2 parts by weight |
| (3) Compound No. 5 | 1 part by weight |

The above-mentioned components are mixed together and pulverized to form a dust.

Formulation Example 2

| | |
|---|---|
| (1) Compound No. 1 | 75 parts by weight |
| (2) a polycarboxylic acid type polymer (trade name: Demol EP powder) | 13.5 parts by weight |
| (3) NaCl | 10 parts by weight |
| (4) dextrin | 0.5 part by weight |
| (5) an alkyl sulfonate (trade name: TP-89121) | 1 part by weight |

The above-mentioned components are placed in a high-speed mixing granulator, admixed with 20 wt% of water, granulated, and dried to form water-dispersible granules.

Formulation Example 3

| | |
|---|---|
| (1) kaolin | 78 parts by weight |
| (2) a condensate of sodium naphthalenesulfonate and formalin (trade name: Lavelin FAN) | 2 parts by weight |
| (3) a sodium polyoxyethylene alkylaryl ether sulfate premixed with white carbon (Trade name: Sorpol 5039) | 5 parts by weight |
| (4) white carbon (trade name: Carplex) | 15 parts by weight |

A mixture of the above-mentioned components (1) to (4) is mixed with Compound No. 54 at a weight ratio of 9:1 to form a wettable powder.

Formulation Example 4

| | |
|---|---|
| (1) diatomaceous earth | 63 parts by weight |
| (2) a polyoxyethylene alkylphenyl ether sulfate ammonium salt premixed with white carbon (trade name: Dikssol W-66) | 5 parts by weight |
| (3) a dialkyl sulfosuccinate premixed with white carbon (trade name: Dikssol W-09B) | 2 parts by weight |
| (4) Compound No. 2 | 30 parts by weight |

The above-mentioned components are mixed together to form a wettable powder.

Formulation Example 5

| | |
|---|---|
| (1) talc micropowder (trade name: Hi-Filler No. 10) | 33 parts by weight |
| (2) a dialkyl sulfosuccinate premixed with white carbon (trade name: Sorpol 5050) | 3 parts by weight |
| (3) a mixture of a polyoxyethylene alkylarylether sulfate and a polyoxyethylene monomethyl ether carbonate, premixed with white carbon (trade name: Sorpol 5073) | 4 parts by weight |
| (4) Compound No. 3 | 60 parts by weight |

The above-mentioned components are mixed together to form a wettable powder.

Formulation Example 6

| | |
|---|---|
| (1) Compound No. 4 | 4 parts by weight |
| (2) corn oil | 79 parts by weight |
| (3) a mixture of dialkyl sulfosuccinate, polyoxyethylene nonylphenyl ether, polyoxyethylene hydrogenated castor oil and polyglycerol esters of fatty acid (trade name: Sorpol 3815) | 15 parts by weight |
| (4) a bentonite-alkylamino complex (trade name: New D Orben) | 2 parts by weight |

The above-mentioned components are uniformly mixed together and pulverized with a Dyno mill (manufactured by Willy A. Bachofen) to form a suspension concentrate.

While the invention has been described in detail and with reference to specific embodiments thereof, it will be apparent to one skilled in the art that various changes and modifications can be made therein without departing from the spirit and scope thereof.

## Claims

1. A substituted pyridinesulfonamide compound or its salt represented by the following general formula (I):

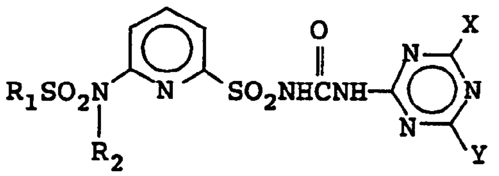

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups.

2. The compound or the salt thereof as in claim 1, wherein X and Y are each independently a member selected from the group consisting of methyl group and methoxy group.

3. The compound or the salt thereof as in claim 1, wherein $R_1$ and $R_2$ may be either each independently alkyl groups which include those having 2 to 4 carbon atoms, and X and Y are each independently a member selected from the group consisting of a methyl group and a methoxy group.

4. The compound or the salt thereof as in claim 1, which is 6-[(N-ethyl-N-isopropylsulfonyl)amino]-N-[[(4-methoxy-6-methyltriazin-2-yl)amino]carbonyl]-2-pyridinesulfonamide, 6-[(N-ethyl-N-isopropylsulfonyl)amino]-N-[[(4,6-dimethoxytriazin-2-yl)amino]carbonyl]-2-pyridinesulfonamide, 6-[[N-(1-chloroethylsulfonyl)-N-ethyl]amino]-N-[[(4-methoxy-6-methyltriazin-2-yl)amino]carbonyl]-2-pyridinesulfonamide, 6-[[N-(1-chloroethylsulfonyl)-N-ethyl]amino]-N-[[(4,6-dimethoxytriazin-2-yl)amino]-carbonyl]-2-pyridinesulfonamide or 6-[(N-dichloromethylsulfonyl-N-ethyl)amino]-N-[[(4,6-dimethoxytriazine-2-yl)amino]carbonyl]-2-pyridinesulfonamide.

5. The compound or the salt thereof as in claim 1, which is 6-[[N-(1-chloroethylsulfonyl)-N-ethyl]amino]-N-[[(4-methoxy-6-methyltriazin-2-yl)amino]carbonyl]-2-pyridinesulfonamide.

6. A herbicide containing as the effective ingredient a substituted pyridinesulfonamide compound or its salt represented by the following general formula (I):

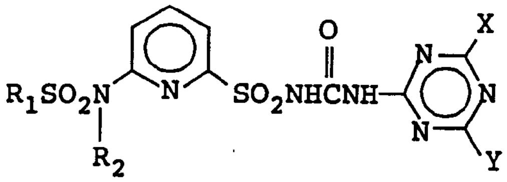

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups.

7. The herbicide as in claim 6, wherein X and Y are each independently a member selected from the group consisting of methyl group and methoxy group.

8. The herbicide as in claim 6, wherein $R_1$ and $R_2$ may be either each independently alkyl groups which include those having 2 to 4 carbon atoms, and X and Y are each independently a member selected from the group consisting of methyl group and methoxy group.

9. The herbicide as in claim 6, wherein said effective ingredient is 6-[(N-ethyl-N-isopropylsulfonyl)amino]-N-[[(4-methoxy-6-methyltriazin-2-yl)amino]carbonyl]-2-pyridinesulfonamide, 6-[(N-ethyl-N-isopropylsulfonyl)amino]-N-[[(4,6-dimethoxytriazin-2-yl)amino]carbonyl]-2-pyridinesulfonamide, 6-[[N-(1-chloroethylsulfonyl)-N-ethyl]amino]-N-[[(4-methoxy-6-methyltriazin-2-yl)amino]carbonyl]-2-pyridinesulfonamide, 6-[[N-(1-chloroethylsulfonyl)-N-ethyl]amino]-N-[[(4,6-dimethoxytriazin-2-yl)amino]carbonyl]-2-pyridinesulfonamide or 6-[(N-dichloromethylsulfonyl-N-ethyl)amino]-N-[[(4,6-dimethoxytriazine-2-yl)amino]carbonyl]-2-pyridinesulfonamide.

10. The herbicide as in claim 6, wherein said effective ingredient is 6-[[N-(1-chloroethylsulfonyl]-N-ethyl]amino]-N-[[(4-methoxy-6-methyltriazin-2-yl)amino]carbonyl]-2-pyridinesulfonamide.

11. A herbicidal composition which comprises:

a herbicidally effective amount of at least one compound selected from the group consisting of substituted pyridinesulfonamide compounds or their salts represented by the following general formula (I):

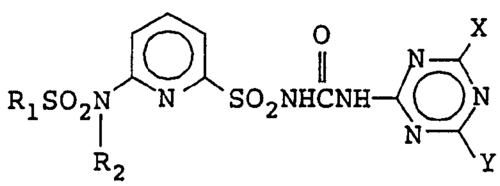

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups;

a herbicidally effective amount of at least one other compound; and

agricultural adjuvants.

12. A herbicidal composition which comprises:

a herbicidally effective amount of at least one compound selected from the group consisting of substituted pyridinesulfonamide compounds or their salts represented by the following general formula (I):

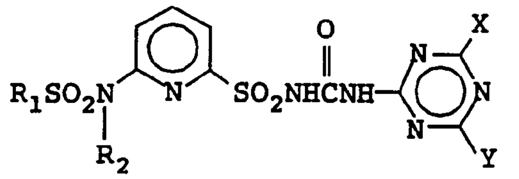

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups;

a herbicidally effective amount of at least one compound selected from the group consisting of ethyl (±)-2-[4-[(6-chloro-2-quinoxalinyl)oxy]phenoxy]propionate, ethyl (±)-2-[4-[(6-chloro-2-benzoxazolyl)oxy] phenoxy]propionate, butyl (±)-2-[4-[[5-(trifluoromethyl)-2-pyridinyl]oxy]phenoxy]propionate, methyl 2-[4-[[3-chloro-5-(trifluoromethyl)-2-pyridinyl]oxy]phenoxy]propionate, 2-ethoxyethyl 2-[4-[[3-chloro-5-(trifluoromethyl)-2-pyridinyl]oxy]phenoxy]propionate, (R)-2-[[(1-methylethylidene)amino]oxy]ethyl 2-[4-[6-chloro-2-quinoxalinyl)oxy]phenoxy]propanoate, sodium 5-[2-chloro-4-(trifluoromethyl)phenoxy]-2-nitrobenzoate, (±)-2-ethoxy-1-methyl-2-oxoethyl-5-[2-chloro-4-(trifluoromethyl)phenoxy]-2-nitrobenzoate, 5-[2-chloro-4-(trifluoromethyl)phenoxy]-N-(methylsulfonyl)-2-nitrobenzamide, 2-chloro-N-(2-ethyl-6-methylphenyl)-N-(2-methoxy-1-methylethyl)acetamide, 2-chloro-N-(2,6-diethylphenyl)-N-(methoxymethyl)acetamide, N-[2,4-dimethyl-5-[[(trifluoromethyl)sulfonyl]amino]phenyl]acetamide, (±)-2-[4,5-dihydro-4-methyl-4-(1-methylethyl)-5-oxo-1H-imidazol-2-yl]-5-ethyl-3-pyridinecarboxylic acid, 2-[4,5-dihydro-4-methyl-4-(1-methylethyl)-5-oxo-1H-imidazol-2-yl]-3-quinolinecarboxylic acid, 2,6-dinitro-N,N-dipropyl-4-(trifluoromethyl)aniline, N-(1-ethylpropyl)-3,4-dimethyl-2,6-dinitroaniline, N-ethyl-α,α,α-tri-fluoro-N-(2-methylallyl)-2,6-dinitro-p-toluidine, 3-[(methoxycarbonyl)amino]phenyl (3-methylphenyl)carbamate, ethyl [3-[[(phenylamino)carbonyl]oxy]phenyl]carbamate, S-ethyl dipropylcarbamothioate, S-propyl dipropylthiocarbamate, 2-[1-(ethoxyimino)butyl]-5-[2-(ethylthio)propyl]-3-hydroxy-2-cyclohexen-1-one, ethyl 2-[[[[(4-chloro-6-methoxy-2-pyrimidinyl)amino]carbonyl]amino]sulfonyl]benzoate, methyl 3-[[[[(4-methoxy-6-methyl-1,3,5-triazin-2-yl)amino]carbonyl]amino]sulfonyl]-2-thiophenecarboxylate, 3-(1-methylethyl)-(1H)-2,1,3-benzothiadiazin-4(3H)-one-2,2-dioxide, 4-(2,4-dichlorophenoxy)butanoic acid, 3-(3,4-dichlorophenyl)-1-methoxy-1-methylurea, 4-amino-6-(1,1-dimethylethyl)-3-(methylthio)-1,2,4-triazin-5(4H)-one, 7-oxabicyclo-[2.2.1]heptane-2,3-dicarboxylic acid, (±)-2-ethoxy-2,3-dihydro-3,3-dimethyl-5-benzofuranyl methanesulfonate, 5-amino-4-chloro-2-phenyl-3(2H)-pyridazinone, 4-chloro-5-methylamino-2-(α,α,α-trifluoro-m-tolyl)pyridazin-3(2H)-one and 2-(2-chlorobenzyl)-4,4-dimethyl-1,2-oxazolidin-3-one; and

agricultural adjuvants.

**13.** The herbicidal composition as in claim 12, wherein X and Y in formula (I) each represents a methyl group or a methoxy group.

**14.** The herbicidal composition as in claim 12, wherein $R_1$ and $R_2$ in formula (I) each represents an alkyl group having from 2 to 4 carbon atoms; and X and Y each represents a methyl group or a methoxy group.

**15.** A herbicidal composition which comprises:

a herbicidally effective amount of at least one compound selected from the group consisting of substituted pyridinesulfonamide compounds or their salts represented by the following general formula (I):

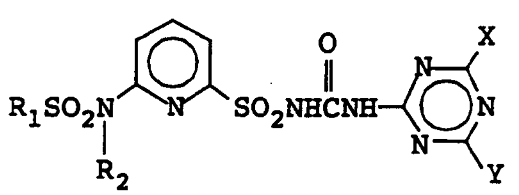

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups;

a herbicidally effective amount of at least one compound selected from the group consisting of methyl 2-[[[[(4-methoxy-6-methyl-1,3,5-triazin-2-yl)amino]-carbonyl]amino]sulfonyl]benzoate, methyl 2-[[[[N-(4-methoxy-6-methyl-1,3,5-triazin-2-yl)methylamino]-carbonyl]amino]sulfonyl]benzoate, 2-chloro-N-[[(4-methoxy-6-methyl-1,3,5-triazin-2-yl]amino]carbonyl]-benzenesulfonamide, methyl (±)-2-[4-(2,4-dichlorophenoxy)phenoxy]propionate, methyl 6-(4-isopropyl-4-methyl-5-oxo-2-imidazolin-2-yl)-m-toluate, methyl 2-(4-isopropyl-4-methyl-5-oxo-2-imidazolin-2-yl)-p-toluate, 1,2-dimethyl-3,5-diphenyl-1H-pyrazolium methylsulfate, 3,5-dibromo-4-hydroxybenzonitrile, 4-hydroxy-3,5-diiodobenzonitrile, 2,4-dichlorophenoxyacetic acid, S-(2,3,3-trichloro-2-propenyl)-bis(1-methylethyl)carbamothioate, 4-chloro-2-methylphenoxyacetic acid, 3,6-dichloro-2-pyridinecarboxylic acid, O-(6-chloro-3-phenyl-4-pyridazinyl)-S-octyl thiocarbonothioate, 3,6-dichloro-2-methoxybenzoic acid, N'-(3,4-dichlorophenyl)-N,N-dimethylurea and 4-amino-3,5,6-trichloro-2-pyridinecarboxylic acid; and

agricultural adjuvants.

**16.** A herbicidal composition which comprises:

a herbicidally effective amount of at least one compound selected from the group consisting of substituted pyridinesulfonamide compounds or their salts represented by the following general formula (I):

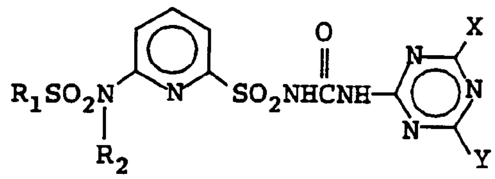

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups;

a herbicidally effective amount of at least one compound selected from the group consisting of N,N-dimethyl N'-[3-(trifluoromethyl)phenyl]urea, 2-[[4-chloro-6-(ethylamino)-1,3,5-triazin-2-yl]amino]-2-methylpropionitrile, N,N'-bis(1-methylethyl)-6-(methylthio)-1,3,5-triazine-2,4-diamine, N-(3,4-dichlorophenyl)propanamide, disodium salt of methylarsonic acid, monosodium salt of methylarsonic acid, 2-chloro-1-(3-ethoxy-4-nitrophenoxy)-4-(trifluoromethyl)benzene, O,O-bis(1-methylethyl) S-[2-[(phenylsulfonyl)amino]ethyl]phosphorodithioate, 2-(3,4-dichlorophenyl)-4-methyl-1,2,4-oxadiazolidine-3,5-dione, 4-chloro-5-(methylamino)-2-[3-(trifluoromethyl)phenyl]-3(2H)-pyridazinone, 2-chloro-6-(4,6-dimethoxypyrimidin-2-ylthio)benzoate, ethyl 2-chloro-6-(4,6-dimethoxypyrimidin-2-ylthio)benzoate and sodium 2-chloro-6-(4,6-dimethoxypyrimidin-2-ylthio)benzoate; and

agricultural adjuvants.

**17.** A herbicidal method which comprises applying to plants a herbicidally effective amount of a substituted pyridinesulfonamide compound or its salt represented by the following general formula (I):

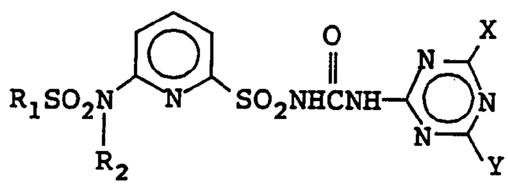

(I)

wherein $R_1$ is an unsubstituted or substituted alkyl groups; $R_2$ is an unsubstituted or substituted alkyl group, or an unsubstituted or substituted alkoxy group; and X and Y are each independently a member selected from the group consisting of alkyl groups and alkoxy groups.

**18.** A process for preparing a substituted pyridinesulfonamide compound or its salt represented by the following general formula (I):

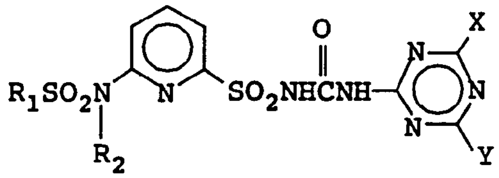

(I)

wherein $R_1$ is an unsubstituted or substituted alkyl group; $R_2$ is an unsubstituted or substituted alkyl group, or an unsubstituted or substituted alkoxy group; and X and Y are each independently a member selected from the group consisting of alkyl groups and alkoxy groups,
which comprises reacting a substituted pyridine compound represented by the following general formula (II):

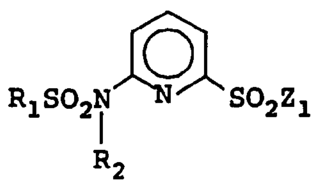

(II)

wherein $R_1$ and $R_2$ are the same as defined above; and $Z_1$ is a member selected from the group consisting of an $-NH_2$ group, an -NCO group, and $-NHCO_2R_3$ groups wherein $R_3$ is an alkyl or aryl group;
with a triazin compound represented by the following general formula (III):

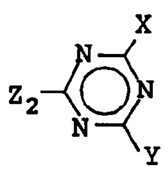

(III)

wherein X and Y are the same as defined above; and $Z_2$ is an $-NH_2$ group when $Z_1$ is an -NCO group or an $-NHCO_2R_3$ group, and is a member selected from the group consisting of an -NCO group and $-NHCO_2R_3$ groups wherein $R_3$ is the same as defined above, when $Z_1$ is an $-NH_2$ group.

**19.** A compound represented by formula:

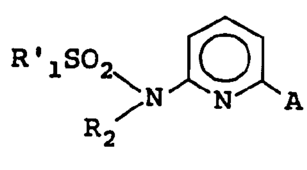

wherein $R'_1$ represents an alkyl group substituted with a halogen atom; $R_2$ represents a substituted or unsubstituted alkyl group or a substituted or unsubstituted alkoxy group; and A represents $-SO_2NH_2$ or

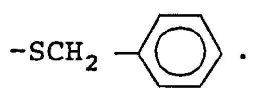

**20.** The compound as in claim 19, wherein $R'_1$ represents $-CH(Cl)CH_3$; $R_2$ represents an ethyl group; and A

represents $-SO_2NH_2$.

**21.** The compound as in claim 19, wherein $R'_1$ represents $-CH(Cl)CH_3$; $R_2$ represents an ethyl group; and A represents

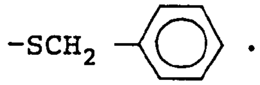

**22.** The compound as in claim 19, werein $R'_1$ represents $-CHCl_2$; $R_2$ represents an ethyl group; and A represents $-SO_2NH_2$.

**23.** The compound as in claim 19, werein $R'_1$ represents $-CHCl_2$; $R_2$ represents an ethyl group; and A represents

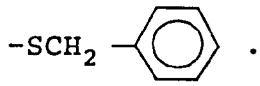

European Patent Office

# EUROPEAN SEARCH REPORT

Application Number
EP 93 30 1705

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,Y | EP-A-0 496 608 (ISHIHARA SANGKYO KAISHA, LTD.)<br>29 July 1992<br>* examples *<br>* claims 1-18 * | 1-18, 20-23 | C07D401/12<br>A01N47/36<br>C07D213/76 |
| P,X | * table 1; intermediate nos. 5-10,25-27 * | 19 | |
| P,Y | PATENT ABSTRACTS OF JAPAN<br>vol. 017, no. 042 (C-1020)26 January 1993<br>& JP-A-42 57 580 ( ISHIHARA SANGYO KAISHA LTD. ) 11 September 1992<br>* abstract * | 1-23 | |
| P,Y | PATENT ABSTRACTS OF JAPAN<br>vol. 017, no. 037 (C-1019)25 January 1993<br>& JP-A-42 53 974 ( ISHIHARA SANGYO KAISHA LTD ) 9 September 1992<br>* abstract * | 1-23 | |
| D,Y | EP-A-0 451 468 (ISHIHARA SANGYO KAISHA, LTD.)<br>16 October 1991<br>* entire document *<br>* claims 1-18 * | 1-23 | TECHNICAL FIELDS SEARCHED (Int. Cl.5)<br>C07D<br>A01N |
| Y | EP-A-0 314 505 (E.I. DU PONT DE NEMOURS AND COMPANY)<br>3 May 1989<br>* entire document *<br>* claims 1-16 * | 1-23 | |
| D,Y | US-A-4 946 494 (E.I. DU PONT DE NEMOURS AND COMPANY)<br>7 August 1990<br>* entire document *<br>* claims 1-15 * | 1-23 | |

The present search report has been drawn up for all claims

| Place of search | Date of completion of the search | Examiner |
|---|---|---|
| MUNICH | 14 JUNE 1993 | HERZ C.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)